# EUROPEAN PATENT SPECIFICATION

(11) **EP 0 706 521 B1**
(45) Date of publication and mention of the grant of the patent: **02.10.2002**
(21) Application number: 94921439.9
(22) Date of filing: 01.07.1994
(51) Int. Cl.: C07D 401/04, C07D 471/04, C07D 403/06, A61K 31/33

(54) **IMIDES AS TNF ALPHA INHIBITORS**
IMIDE ALS TNF ALPHA INHIBITOREN
INHIBITEURS DU TNF ALPHA

(30) Priority: 02.07.1993 US 87510
(43) Date of publication of application: 17.04.1996
(60) Divisional application: 00200492.7
(73) Proprietor: CELGENE CORPORATION, Warren New Jersey 07059 (US)
(72) Inventor: MULLER, George, W., Bridgewater, nJ (US)
(74) Representative: Howard, Paul Nicholas
(86) International application number: PCT/US94/07411
(87) International publication number: WO 95/001348

(56) References cited:
- WO-A-92/14455
- GB-A- 768 821
- GB-A- 1 030 030
- GB-A- 1 036 694
- US-A- 3 553 217
- US-A- 4 918 193
- JOURNAL OF PHARMACEUTICAL SCIENCES, vol.57, no.5 pages 757 - 764 SHEALY Y.F. ET AL. 'Synthesis of D- and L-thalidomide and related studies'
- JOURNAL OF THE AMERICAN CHEMICAL SOCIETY, vol.82, 5 September 1960, WASHINGTON, DC US pages 4596 - 4600 R. PAUL ET AL. 'N,N'-Carbonyldiimidazole, a new peptide forming reagent.'
- CHEMICAL ABSTRACTS, vol. 72, no. 17, 27 April 1970, Columbus, Ohio, US; abstract no. 90878f, KWIZDA,F. JOH. 'Cyclic derivatives of succinic and glutaric acids.' & FR,A,1 570 452 (CHEMISCHE FABRIK)
- CHEMICAL ABSTRACTS, vol. 118, no. 11, 15 March 1993, Columbus, Ohio, US; abstract no. 102407k, XU, WENFANG ET AL. 'Synthesis and antitumor activity of glutamine derivatives' & ZHONNNUO YIYAO GONGYE ZAZHI, vol.23, no.6, 1992 pages 255 - 258
- CHEMICAL ABSTRACTS, vol. 87, no. 7, 15 August 1977, Columbus, Ohio, US; abstract no. 52897p, ABO-SIER,A.H. ET AL. 'Synthesis of ...' & PHARMAZIE, vol.32, no.3, 1977 pages 149 - 150
- CHEMICAL ABSTRACTS, vol. 78, no. 7, 19 February 1973, Columbus, Ohio, US; abstract no. 43414r, JONSSON,N.A. 'Chemical structure ...' & ACTA PHARM. SUEC., vol.9, no.5, 1972 pages 431 - 446
- CHEMICAL ABSTRACTS, vol. 105, no. 17, 27 October 1986, Columbus, Ohio, US; abstract no. 145893q, OWEN,D.A. 'Pharmacological ...' & CARDIOVASC. PHARMACOL., vol.8, no.4, 1986 pages 743 - 748
- CHEMICAL ABSTRACTS, vol. 106, no. 21, 25 May 1987, Columbus, Ohio, US; abstract no. 168374f, CZEJKA, MARTIN J. ET AL. 'Determination ...' & J. CHROMATOGR., vol.413, 1987 pages 181 - 187
- CHEMICAL ABSTRACTS, vol. 94, no. 19, 11 May 1981, Columbus, Ohio, US; abstract no. 151262d, FLOHE,L. ET AL. 'Studies ...' & ARZNEIM.-FORSCH., vol.31, no.2, 1981 pages 315 - 320
- CHEMICAL ABSTRACTS, vol. 121, no. 23, 5 December 1994, Columbus, Ohio, US; abstract no. 280507n, HUANG,JUNQIN ET AL. 'Chemical ...' & ZHONGGUO YIYAO GONGYE ZAZHI, vol.24, no.10, 1993 pages 437 - 441
- CHEMICAL ABSTRACTS, vol. 91, no. 13, 24 September 1979, Columbus, Ohio, US; abstract no. 107613h, DE, A.U. ET AL. 'Prototype ...' & INDIAN J, CHEM., SECT. B, vol.17, no.B1, 1079 pages 57 - 61
- CHEMICAL ABSTRACTS, vol. 86, no. 23, 6 June 1977, Columbus, Ohio, US; abstract no. 170837w, DE, A. U. ET AL. 'Possible ...' & J. PHARM. SCI., vol.66, no.2, 1977 pages 232 - 235
- J.Med.Chem., 39, 1996, pp. 3238-3240
- Chemtech., 27(1), 1997, pp. 21-25
- Molecular Medicine, 2(4), 1996, pp. 506-515
- Bioorg.Med.Chem.Lett., 8, 1998, pp. 2669-2674

## Description

The present invention relates to compounds and compositions which prevent or inhibit the production or action of TNF-α.

TNF_{α}, or tumor necrosis factor α, is a cytokine which is released primarily by mononuclear phagocytes in response to various immunostimulators. When administered to animals or humans it causes inflammation, fever, cardiovascular effects, hemorrhage, coagulation and acute phase responses similar to those seen during acute infections and shock states.

Excessive or unregulated TNF_{α} production has been implicated in a number of disease conditions. These include endo-toxemia and/or toxic shock syndrome {Tracey et al., Nature **330,** 662-664 (1987) and Hinshaw et al., Circ. Shock **30,**279-292 (1990)}; cachexia {Dezube et al., Lancet, **335** (8690), 662 (1990)}; and Adult Respiratory Distress Syndrome where TNF_{α} concentration in excess of 12,000 pg/mL have been detected in pulmonary aspirates from ARDS patients (Millar et al., Lancet **2**(8665), 712-714 (1989)}. Systemic infusion of recombinant TNF_{α} also resulted in changes typically seen in ARDS (Ferrai-Baliviera et al., Arch. Surg. **124**(12), 1400-1405 (1989)}.

TNF_{α} appears to be involved in bone resorption diseases, including arthritis where it has been determined that when activated, leukocytes will produce a bone-resorbing activity, and data suggest that TNF_{α} contributes to this activity. (Bertolini et al., Nature **319,** 516-518 (1986) and Johnson et al., Endocrinology **124**(3), 1424-1427 (1989).} It has been determined that TNF_{α} stimulates bone resorption and inhibits bone formation in vitro and in vivo through stimulation of osteoclast formation and activation combined with inhibition of osteoblast function. Although TNF_{α} may be involved in many bone resorption diseases, including arthritis, the most compelling link with disease is the association between production of TNF_{α} by tumor or host tissues and malignancy associated hypercalcemia {Calci. Tissue Int. (US) **46**(Suppl.), S3-10 (1990)}. In Graft versus Host Reaction, increased serum TNF_{α} levels have been associated with major complication following acute allogenic bone marrow transplants {Holler et al., Blood, **75**(4), 1011-1016 (1990)}.

Cerebral malaria is a lethal hyperacute neurological syndrome associated with high blood levels of TNF_{α} and the most severe complication occurring in malaria patients. Levels of serum TNF_{α} correlated directly with the severity of disease and the prognosis in patients with acute malaria attacks {Grau et al., N. Engl. J. Med. **320**(24), 1586-1591 (1989)}.

TNF_{α} also plays a role in the area of chronic pulmonary inflammatory diseases. The deposition of silica particles leads to silicosis, a disease of progressive respiratory failure caused by a fibrotic reaction. Antibody to TNF_{α} completely blocked the silica-induced lung fibrosis in mice {Pignet et al., Nature, **344**:245-247 (1990)}. High levels of TNF_{α} production (in the serum and in isolated macrophages) have been demonstrated in animal models of silica and asbestos induced fibrosis {Bissonnette et al., Inflammation **13**(3), 329-339 (1989)}. Alveolar macrophages from pulmonary sarcoidosis patients have also been found to spontaneously release massive quantities of TNF_{α} as compared with macrophages from normal donors {Baughman et al., J. Lab. Clin. Med. **115**(1), 36-42 (1990)).

TNF_{α} is also implicated in the inflammatory response which follows reperfusion, called reperfusion injury, and is a major cause of tissue damage after loss of blood flow (Vedder et al., PNAS **87**, 2643-2646 (1990)). TNF_{α} also alters the properties of endothelial cells and has various pro-coagulant activities, such as producing an increase in tissue factor pro-coagulant activity and suppression of the anticoagulant protein C pathway as well as down-regulating the expression of thrombomodulin {Sherry et al., J. Cell Biol. **107,** 1269-1277 (1988)). TNF_{α} has pro-inflammatory activities which together with its early production (during the initial stage of an inflammatory event) make it a likely mediator of tissue injury in several important disorders including but not limited to, myocardial infarction, stroke and circulatory shock. Of specific importance may be TNF_{α}-induced expression of adhesion molecules, such as intercellular adhesion molecule (ICAM) or endothelial leukocyte adhesion molecule (ELAM) on endothelial cells {Munro et al., Am. J. Path. **135**(1), 121-132 (1989)}.

Moreover, it now is known that TNF_{α} is a potent activator of retrovirus replication including activation of HIV-1. (Duh et al., Proc. Nat. Acad. Sci. **86**, 5974-5978 (1989); Poll et al., Proc. Nat. Acad. Sci. **87**, 782-785 (1990); Monto et al., Blood **79**, 2670 (1990); Clouse et al., J. Immunol. **142**, 431-438 (1989); Poll et al., AIDS Res. Hum. Retrovirus, 191-197 (1992)). AIDS results from the infection of T lymphocytes with Human Immunodeficiency Virus (HIV). At least three types or strains of HIV have been identified, i.e., HIV-1, HIV-2 and HIV-3. As a consequence of HIV infection, T-cell mediated immunity is impaired and infected individuals manifest severe opportunistic infections and/or unusual neoplasms. HIV entry into the T lymphocyte requires T lymphocyte activation. Other viruses, such as HIV-1, HIV-2 infect T lymphocytes after T cell activation and such virus protein expression and/or replication is mediated or maintained by such T cell activation. Once an activated T lymphocyte is infected with HIV, the T lymphocyte must continue to be maintained in an activated state to permit HIV gene expression and/or HIV replication. Cytokines, specifically TNF_{α}, are implicated in activated T-cell mediated HIV protein expression and/or virus replication by playing a role in maintaining T lymphocyte activation. Therefore, interference with cytokine activity such as by prevention or inhibition of cytokine production, notably TNF_{α}, in an HIV-infected individual aids in limiting the maintenance of T lymphocyte caused by HIV infection.

Monocytes, macrophages, and related cells, such as kupffer and glial cells, have also been implicated in maintenance of the HIV infection. These cells, like T cells, are targets for viral replication and the level of viral replication is dependent upon the activation state of the cells. (Rosenberg et al., The Immunopathogenesis of HIV Infection, Advances in Immunology, **57** (1989)}. Cytokines, such as TNF_{α}, have been shown to activate HIV replication in monocytes and/or macrophages {Poli et al. Proc. Natl. Acad. Sci., **87**, 782-784 (1990)}, therefore, prevention or inhibition of cytokine production or activity aids in limiting HIV progression as stated above for T cells. Additional studies have identified TNF_{α} as a common factor in the activation of HIV in vitro and has provided a clear mechanism of action via a nuclear regulatory protein found in the cytoplasm of cells (Osborn, et al., PNAS **86** 2336-2340). This evidence suggests that a reduction of TNF_{α} synthesis may have an antiviral effect in HIV infections, by reducing the transcription and thus virus production.

AIDS viral replication of latent HIV in T cell and macrophage lines can be induced by TNF_{α} {Folks et al., PNAS **86**, 2365-2368 (1989)}. A molecular mechanism for the virus inducing activity is suggested by TNF_{α}'s ability to activate a gene regulatory protein (NFKB) found in the cytoplasm of cells, which promotes HIV replication through binding to a viral regulatory gene sequence (LTR) {Osborn et al., PNAS **86,** 2336-2340 (1989)}. TNF_{α} in AIDS associated cachexia is suggested by elevated serum TNF_{α} and high levels of spontaneous TNF_{α} production in peripheral blood monocytes from patients {Wright et al. J. Immunol. **141**(1), 99-104 (1988)}.

TNF_{α} has been implicated in various roles with other viral infections, such as the cytomegalia virus (CMV), influenza virus, adenovirus, and the herpes family of viruses for similar reasons as those noted.

Preventing or inhibiting the production or action of TNF_{α} is, therefore, predicted to be a potent therapeutic strategy for many inflammatory, infectious, immunological or malignant diseases. These include but are not restricted to septic shock, sepsis, endotoxic shock, hemodynamic shock and sepsis syndrome, post ischemic reperfusion injury, malaria, mycobacterial infection, meningitis, psoriasis, congestive heart failure, fibrotic disease, cachexia, graft rejection, cancer, autoimmune disease, opportunistic infections in AIDS, rheumatoid arthritis, rheumatoid spondylitis, osteoarthritis, other arthritic conditions, Crohn's disease, ulcerative colitis, multiple sclerosis, systemic lupus erythrematosis, ENL in leprosy, radiation damage, and hyperoxic alveolar injury. Efforts directed to the suppression of the effects of TNF_{α} have ranged from the utilization of steroids such as dexamethasone and prednisolone to the use of both polyclonal and monoclonal antibodies (Beutler et al., Science **234**, 470-474 (1985); WO 92/11383).

The nuclear factor kB (NFKB) is a pleiotropic transcriptional activator (Lenardo, et al. Cell 1989, 58, 227-29). NFKB has been implicated as a transcriptional activator in a variety of disease and inflammatory states and is thought to regulate cytokine levels including but not limited to TNF_{α} and also to be an activator of HIV transcription (Dbaibo, et al. J. Biol. Chem. 1993, 17762-66; Duh et al. Proc. Natl. Acad. Sci. 1989, 86, 5974-78; Bachelerie et al. Nature 1991, 350, 709-12; Boswas et al. J.. Acquired Immune Deficiency Syndrome 1993, 6, 778-786; Suzuki et al. Biochem. And Biophys. Res. Comm. 1993, 193, 277-83; Suzuki et al. Biochem. And Biophys. Res Comm. 1992, 189, 1709-15; Suzuki et al. Biochem. Mol. Bio. Int. 1993, 31(4), 693-700; Shakhov et al. 1990, 171, 35-47; and Staal et al. Proc. Natl. Acad. Sci. USA 1990, 87, 9943-47). Thus, inhibition of NFKB binding can regulate transcription of cytokine gene(s) and through this modulation and other mechanisms be useful in the inhibition of a multitude of disease states. The compounds claimed in this patent can inhibit the action of NFKB in the nucleus and thus are useful in the treatment of a variety of diseases including but not limited to rheumatoid arthritis, rheumatoid spondylitis, osteoarthritis, other arthritic conditions, septic shock, septis, endotoxic shock, graft versus host disease, wasting Crohn's disease, ulcerative colitis, multiple sclerosis, systemic lupus erythrematosis, ENL in leprosy, HIV, AIDS, and opportunistic infections in AIDS.

TNF_{α} and NFKB levels are influenced by a reciprocal feedback loop. As noted above, the compounds of the present invention affect the levels of both TNF_{α} and NFKB. It is not known at this time, however, how the compounds of the present invention regulate the levels of TNF_{α}, NFKB, or both.

United States Patent 3,553,217 describes preparation of the chemical compound thalidomide and its use as a sedative. In addition, WO92/1445 discloses use of thalidomide, as well as certain derivatives and hydrolysis products thereof, as useful in the control of TNFa. United Kingdom Patent 1,036,694 describes various substituted aminocarboxylic acid derivatives as having activity in the CNS (central nervous system) and United kingdom Patent 1,030,030 describes various phthalimide derivatives as possessing tranquillizing and anticonvulsant activity.

### Detailed Description

The present invention is based on the discovery that a class of non-polypeptide imides more fully described herein appear to inhibit the action of TNF_{α}.

A first aspect of the present invention pertains to compounds of the formula: 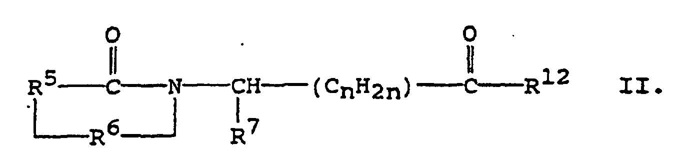 in which R⁵ is (i) o-phenylene, unsubstituted or substituted with 1 to 3 substituents each selected independently from nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, or (ii) the divalent residue of pyridine, pyrrolidine, imidizole, naphthalene, or thiophene, wherein the divalent bonds are on vicinal ring carbon atoms;
R⁶ is -CO-, -CH₂- or -SO₂-;
R⁷ is (i) pyridyl, (ii) phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, (iii) benzyl substituted with 1 to 3 substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, (iv) napthyl, (v) benzyloxy, or (vi) imidazol-4-ylmethyl;
R¹² is -OH, alkoxy of 1 to 12 carbon atoms, -O-CH₂-pyridyl, -O-benzyl or 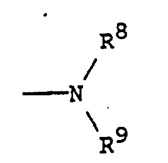 n has a value of 0, 1, 2, or 3;
R⁸ is hydrogen or alkyl of 1 to 10 carbon atoms; and
R⁹ is hydrogen, alkyl of 1 to 10 carbon atoms, -CH₂-pyridyl, benzyl, -COR¹⁰, or -SO₂R¹⁰ in which R¹⁰ is hydrogen, alkyl of 1 to 4 carbon atoms, or phenyl.

A first preferred subclass of Formula II pertains to compounds of the formula: 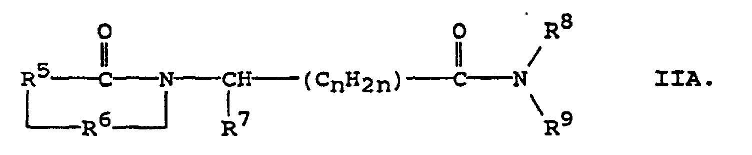 in which R⁵ is (i) o-phenylene, unsubstituted or substituted with 1 to 3 substituents each selected independently from nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, or (ii) the divalent residue of pyridine, pyrrolidine, imidizole, naphthalene, or thiophene, wherein the divalent bonds are on vicinal ring carbon atoms;
R⁶ is -CO-, -CH₂- or -SO₂-;
R⁷ is (i) pyridyl, (ii) phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, (iv) benzyl substituted with 1 to 3 substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, (v) napthyl, (vi) benzyloxy, or (vii) imidazol-4-ylmethyl;
n has a value of 0, 1, 2, or 3; R⁸ is hydrogen or alkyl of 1 to 4 carbon atoms; and R⁹ is hydrogen, alkyl of 1 to 4 carbon atoms, -COR¹⁰, or -SO₂R¹⁰ in which R¹⁰ is hydrogen, alkyl of 1 to 4 carbon atoms, or phenyl.

Preferred compounds of Formula IIA are those in which R⁵ is o-phenylene, R⁶ is -CO-; R⁷ is substituted phenyl or pyridyl; n is 0 or 1, and each of R⁸ and R⁹ is hydrogen.

A second preferred subclass of Formula II pertains to compounds of the formula: 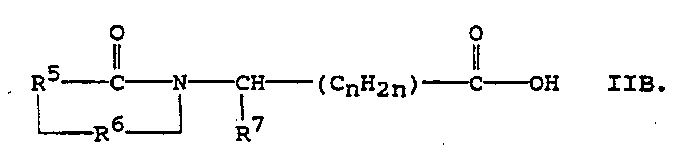 in which R⁵ is (i) o-phenylene, unsubstituted or substituted with 1 to 3 substituents each selected independently from nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, or (ii) the divalent residue of pyridine, pyrrolidine, imidizole, naphthalene, or thiophene, wherein the divalent bonds are on vicinal ring carbon atoms;
R⁶ is -CO-, -CH₂- or -SO₂-;
R⁷ is (i) pyridyl, (ii) phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 14 carbon atoms, or halo, (iii) benzyl substituted with 1 to 3 substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, (iv) napthyl, (v) benzyloxy, or (vi) imidazol-4-ylmethyl; and
n has a value of 0, 1, 2, or 3.

Preferred compounds of Formula IIB are those in which R⁵ is o-phenylene, R⁶ is -CO-; R⁷ is substituted phenyl or pyridyl; and n is 0 or 1.

A third preferred subclass of Formula II pertains to compounds of the formula: 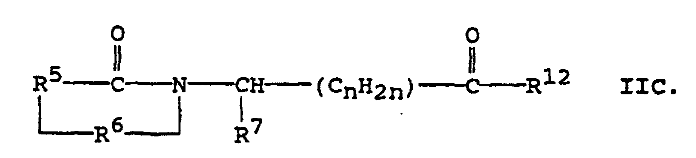 in which R⁵ is (i) o-phenylene, unsubstituted or substituted with 1 to 3 substituents each selected independently from nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, or (ii) the divalent residue of pyridine, pyrrolidine, imidizole, naphthalene, or thiophene, wherein the divalent bonds are on vicinal ring carbon atoms;
R⁶ is -CO-, -CH₂- or -SO₂-;
R⁷ is (i) pyridyl, (ii) phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, (iii) benzyl substituted with 1 to 3 substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, (iv) napthyl, (v) benzyloxy, or (vi) imidazol-4-ylmethyl;
R¹² is -OH or alkoxy of 1 to 12 carbon atoms; and
n has a value of 0, 1, 2, or 3.

Preferred compounds of Formula IIC are those in which R⁵ is o-phenylene or amino substituted o-phenylene; R⁶ is -CO-; R⁷ substituted phenyl or pyridyl; R¹² is methoxy; and n is 0 or 1.

Typical compounds of this invention include 2-phthalimido-3-(4-hydroxy)phenylpropanamide; 2-phthalimido-2-(4-hydroxyphenyl)acetic acid; 2-phthalimido-2-(4-fluorophenyl)acetic acid; 2-phthalimido-2-(2-fluorophenyl)acetic acid; 2-phthalimido-2-(4-fluorophenyl)acetamide; methyl 3-phthalimido-3-(4-methoxyphenyl)propionate; ethyl 3-phthalimido-3-(4-methoxyphenyl)propionate; and propyl 3-phthalimido-3-(4-methoxyphenyl)propionate; 3-(4'-Methoxyphenyl)-3-(1'-oxo-isoindoline)propanoic acid; 3-(4'-Methoxyphenyl)-3-(1'-oxo-isoindoline)propionamide; 3-(3',4'-Dimethoxyphenyl)-3-(1'-oxo-isoindoline)propanoic acid; 3-(3',4'-Dimethoxyphenyl)-3-(1'-oxo-isoindoline)propionamide; 3-(3',4'-Diethoxyphenyl)-3-(1'-oxo-isoindoline)propionic acid; 3-(3',4'-Diethoxyphenyl)-3-phthalimidopropionamide; 3-Phthalimido-3-(4'-propoxyphenyl)propionic acid; 3-Phthalimido-3-(4'-propoxyphenyl) propionamide; Ethyl 3-phthalimido-3-(3'-pyridyl)propionate; 3-Phthalimido-3-(3',4'-dimethoxyphenyl)propionic acid; 3-Phthalimido-3-(3',4'-dimethoxyphenyl)propionamide; Ethyl 3-Amino-3-(3',4'-dimethoxyphenyl)propionate; Ethyl 3-phthalimido-3-(3',4'-dimethoxyphenyl)propionate; 3-Phthalimido-3-(3',4'-dimethoxyphenyl)propionic amylamide; 3-phthalimido-3-(3',4'-dimethoxyphenyl)propionic benzylamide; 3-phthalimido-3-(3',4'-dimethexyphenyl)propionic ethylamide; 3-Phthalimido-3-(4'-ethoxyphenyl)propionic acid; 3-phthalimdo-3-(4'-ethoxyphenyl)propionamide; 3-(4'-nitrophthalimido)-3-(4'-methoxyphenyl)propionic acid; 3-Phthalimido-3-(2'-napthyl)propionic acid; 3-Phthalimido-3-(2'-napthyl)propionamide; Methyl 3-(1,3-dioxo-5-azaisoindol-2-yl)-3-(3',4'-dimethoxyphenyl) -propionate; 3-3-phthalimido-3-(4'-butoxy-3-methoxyphenyl)propionic acid; 3-phthalimido-3-(4'-butoxy-3'-methoxyphenyl)propionamide; 3-(4'-methoxyphenyl)-3-(3'-nitrophthalimido)propionic acid; 3-(4',5'-dichlorophthalimido)-3-(4'-methoxyphenyl)propionic acid; 3-Pyridinemethyl 3-, phthalimido-3-(3',4'-dimethoxyphenyl)propionate N-3-Methylpyridyl 3-phthalimido-3-(3',4'-dimethoxyphenyl)propionamide; 3-Phthalimido-3-(3',4'-dichlorophenyl)propionamide; Methyl 3-phthalimido-3-(3',4'-dimethoxyphenyl)propionate; Methyl (S)-N-Benzyl-N-(R)-a-methylbenzyl-3-(3',4'-dimethoxyphenyl) propionate: Methyl (S)-3-amino-3-(3',4'-dimethoxyphenyl)propionate hydrochloride; Methyl (S)-3-phthalimido-3-(3',4'-dimethoxyphenyl) propionate; Methyl (R)-3-(N-benzyl-N-(S)-a-methylbenzylamino)-3-(3',4'-dimethoxyphenyl)propionate; Methyl (R)-3-amino-3-(3',4'-dimethoxyphenyl)propionate hydrochloride; methyl (3R)-3-phthalimido-3-(3',4'-dimethoxyphenyl)propionate.

The term alkyl as used herein denotes a univalent saturated branched or straight hydrocarbon chain. Unless otherwise stated, such chains can contain from 1 to 18 carbon atoms. Representative of such alkyl groups are methyl, ethyl, propyl, isopropyl, butyl, isobutyl, sec-butyl, tert-butyl, pentyl, isopentyl, neopentyl, tert-pentyl, hexyl, isohexyl, heptyl, octyl, nonyl, decyl, undecyl, dodecyl, tridecyl, tetradecyl, pentadecyl, hexadecyl, heptadecyl, octadecyl, and the like. When qualified by "lower", the alkyl group will contain from 1 to 6 carbon atoms. The same carbon content applies to the parent term "alkane" and to derivative terms such as "alkoxy".

The compounds can be used, under the supervision of qualified professionals, to inhibit the undesirable effects of TNF_{α}. The compounds can be administered orally, rectally, or parenterally, alone or in combination with other therapeutic agents including antibiotics, steroids, etc., to a mammal in need of treatment. Oral dosage forms include tablets, capsules, dragees, and similar shaped, compressed pharmaceutical forms. Isotonic saline solutions containing 20-100 mg/mL can be used for parenteral administration which includes intramuscular, intrathecal, intravenous and intra-arterial routes of administration. Rectal administration can be effected through the use of suppositories formulated from conventional carriers such as cocoa butter.

Dosage regimens must be titrated to the particular indication, the age, weight, and general physical condition of the patient, and the response desired but generally doses will be from about 10 to about 500 mg/day as needed in single or multiple daily administration. In general, an initial treatment regimen can be copied from that known to be effective in interfering with TNF_{α} activity for other TMF_{α} mediated disease states by the compounds of the present invention. Treated individuals will be regularly checked for T cell numbers and T4/T8 ratios and/or measures of viremia such as levels of reverse transcriptase or viral proteins, and/or for progression of cytokine-mediated disease associated problems such as cachexia or muscle degeneration. If no effect is soon following the normal treatment regimen, then the amount of cytokine activity interfering agent administered is increased, e.g., by fifty percent a week.

The compounds of the present invention also can be used topically in the treatment or prophylaxis of topical disease states mediated or exacerbated by excessive TNF_{α} production, respectively, such as viral infections, such as those caused by the herpes viruses, or viral conjunctivitis, etc.

The compounds also can be used in the veterinary treatment of mammals other than humans in need of prevention or inhibition of TNF_{α} production. TNF_{α} mediated diseases for treatment, therapeutically or prophylactically, in animals include disease states such as those noted above, but in particular viral infections. Examples include feline immunodeficiency virus, equine infectious anaemia virus, caprine arthritis virus, visna virus, and maedi virus, as well as other lentiviruses.

Certain of these compounds possess centers of chirality and can exist as optical isomers. Both the racemates of these isomers and the individual isomers themselves, as well as diastereomers when there are two chiral centers, are within the scope of the present invention. The racemates can be used as such or can be separated into their individual isomers mechanically as by chromatography using a chiral absorbant. Alternatively, the individual isomers can be prepared in chiral form or separated chemically from a mixture by forming salts with a chiral acid, such as the individual enantiomers of 10-camphorsulfonic acid, camphoric acid, alpha-bromocamphoric acid, methoxyacetic acid, tartaric acid, diacetyltartaric acid, malic acid, pyrrolidone-5-carboxylic acid, and the like, and then freeing one or both of the resolved bases, optionally repeating the process, so as obtain either or both substantially free of the other; i.e., in a form having an optical purity of >95%.

The compounds can be prepared using methods which are known in general for the preparation of imides. However, the present invention also pertains to an improvement in the formation of the final compounds, as discussed below in greater detail.

An N-alkoxycarbonylimide and an amine thus are allowed to react in the presence of a base such as sodium carbonate or sodium bicarbonate substantially as described by Shealy et al., Chem. & Ind., (1965) 1030-1031) and Shealy et al., J. Pharm. Sci. **57**, 757-764 (1968) to yield the N-substituted imide. Alternatively, a cyclic acid anhydride can be reacted with an appropriate amine to form an imide. Formation of a cyclic imide also can be accomplished by refluxing a solution of an appropriately substituted dicarboxylic acid monoamide in anhydrous tetrahydrofuran with N,N'-carbonyldiimidazole. In contrast to prior art methods which produced a yield of less than 50%, this reaction produces yields in excess of 60%, in some cases greater than 90%. This reaction also has broader applicability, being useful not only in the preparation of compounds of the present invention but also in the preparation of known compounds such as thalidomide.

Prevention or inhibition of production of TNF_{α} by these compounds can be conveniently assayed using anti-TNF_{α} antibodies. For example, plates (Nunc Immunoplates, Roskilde, DK) are treated with 5 µg/mL of purified rabbit anti-TNF_{α} antibodies at 4°C for 12 to 14 hours. The plates then are blocked for 2 hours at 25°C with PBS/0.05% Tween containing 5 mg/mL BSA. After washing, 100 µL of unknowns as well as controls are applied and the plates incubated at 4°C for 12 to 14 hours. The plates are washed and assayed with a conjugate of peroxidase (horseradish) and mouse anti-TNF_{α} monoclonal antibodies, and the color developed with o-phenylenediamine in phosphate-citrate buffer containing 0.012% hydrogen peroxide and read at 492 nm.

The following examples will serve to further typify the nature of this invention but should not be construed as a limitation in the scope thereof, which scope is defined solely by the appended claims.

### EXAMPLE 1

To a stirred mixture of phenylglycine (3.0 g, 20 mmoL) and sodium carbonate (2.23 g, 21 mmoL) in 450 mL of water is added N-carbethoxyphthalimide (4.38 g, 20 mmoL). After 45 minutes, the reaction slurry is filtered. The filtrate is stirred and the pH adjusted to 1-2 with 4 N hydrochloric acid. After 1 hour, the resulting slurry is filtered and the solid washed with water. The solid is dried in vacuo (60°C, <133 Nm⁻² (<1mm)) to afford 2.88 g (51%) of 2-phthalimido-2-phenylacetic acid, which can be alternatively named as N-phthaloylphenylglycine, as a white powder.

Use of β-phenyl-β-alanine, α-phenyl-β-alanine, histidine, and tyrosine in place of phenylglycine in the procedure of this example yields respectively 3-phthalimido-3-phenylpropionic acid, 2-phthalimido-3-phenylpropionic acid, 2-phthalimido-3-imidazolylpropionic acid, and 2-phthalimido-3-(4-hydroxyphenyl)propionic acid.

### EXAMPLE 2

To a stirred mixture of 2-phthalimido-2-phenylacetic acid (2.50 g, 8.89 mmoL) in tetrahydrofuran (50 mL) is added carbonyldiimidazole (1.50 g, 9.25 mmoL) and a few crystals of 4-dimethylaminopyridine. The reaction is then heated to 50°C for 45 minutes. After the reaction mixture cools to room temperature, 1 mL of concentrated ammonium hydroxide is added via syringe. The reaction is stirred for 1 hour, then diluted with 50 mL of water and partially concentrated to remove the majority of the tetrahydrofuran. The resulting slurry is filtered and the solid washed with copious amounts of water. The solid is dried in vacuo (60°C, <133 Nm⁻² (<1mm)) to afford 1.9 g (76%) of 2-phthalimido-2-phenylacetamide, which may be alternatively named as N-phthaloylphenylglycinamide, as an off-white powder: mp 218-220°C; ¹H NMR (DMSO-d₆) δ 9.00-7.75 (m, 4 H, Ar), 7.61 (br s, 1 H, CONH₂), 7.55-7.20 (m, 6 H, Ar, CONH₂), 5.82 (s, 1 H, CHCO₂); ¹³C NMR (DMSO-d₆) δ 168.2, 167.1, 135.6, 134.5, 131.4, 129.4, 127.9, 127.7, 123.1, 56.3. Anal (C₁₆H₁₂N₂O₃), C, H, N.

Use of 3-phthalimido-3-phenylpropionic acid, 2-phthalimido-3-phenylpropionic acid, 2-phthalimido-3-imidazolylpropionic acid, and 2-phthalimido-3-(4-hydroxyphenyl)propionic acid in place of 2-phthalimido-2-phenylacetic acid in the procedure of this example yields respectively 3-phthalimido-3-phenylpropanamide, 2-phthalimido-3-phenylpropanamide, 2-phthalimido-3-imidazolylpropanamide, and 2-phthalimido-3-(4-hydroxy)phenylpropanamide.

### REFERENTIAL EXAMPLE 3

To a stirred solution of 2-phthalimido-3-phenylpropionic acid (2.95 g, 10.0 mmol) in tetrahydrofuran (25 mL) are added carbonyldiimidazole (1.62 g, 10.0 mmol) and a few crystals of 4-N,N-dimethylaminopyridine, followed by 15 mL of tetrahydrofuran. The reaction mixture is stirred at room temperature for 45 minutes and then 1 mL of concentrated ammonium hydroxide is added. After 10 minutes, the reaction mixture is diluted with 50 mL water and the resulting slurry is partially concentrated to remove the tetrahydrofuran and filtered. The solid is washed with water and dried *in vacuo* (60°C, <133Nm⁻² < 1mm)) to afford 2.46 g (84%) of 2-phthalimido-3-phenylpropionamide as a white powder: mp 224-226°C; 1H NMR (DMSO-d₆, 250 MHz) δ 7.79 (s, 4 H, Ar), 7.71 (br s, 1 H, CONH2), 7.32 (br s, 1 H, CONH2), 7.20-7.02 (m. 5H, Ar), 5.06-4.98 (m, 1H), 3.56-3.25 (m, 2H); 13C NMR (DMSO-d₆, 250 MHz) δ: 169.6, 168.0, 137.1, 134.3, 131.2, 129.5, 128.1, 126.3, 122.9, 54.2, 33.7. Anal. Calculated for C₁₇H₁₄N₂O₃. Theoretical: C, 69.38; H,4.79; N, 9.52. Found: C, 69.37; H, 4.73; N, 9.43.

### EXAMPLE 4

To a stirred solution of 4-fluorophenylglycine (3.38 g, 20.0 mmol) and sodium carbonate in 450 mL of 2:1 water:acetonitrile is added N-carbethoxyphthalimide (4.38 g, 20 mmol). After 1 hour, the reaction mixture is partially concentrated to remove the acetonitrile. The resulting slurry is filtered and the pH of the stirred filtrate is adjusted to 1-2 with 4 N hydrochloric acid and then stirred for an additional 30 minutes and filtered. The solid is air-dried and then dried in vacuo (60°C, <133 Nm⁻² (< 1 mm)) to afford 4.55 g (76%) of 2-phthalimido-2-(4-fluorophenyl)acetic acid as a white powder: mp 180-183°C; 1H NMR (DMSO-d₆, 250 MHz) δ 8.10-7.80 (m, 4 H), 7.65-7.45 (m, 4 H), 7.3-7.10 (t, 2 H), 6.10 (s, 1 H); 13C NMR (DMSO-d₆, 250 MHz) δ 168.9, 166.9, 163.6, 159.7, 135.0, 131.4, 131.3 (m), 130.9, 123.5, 115.0, 114.7, 54.4. Anal. Calculated for C₁₆H₁₀NO₄F. Theoretical: C, 64.22; H, 3.37; N, 4.68. Found: C, 64.13; H, 3.33; N, 4.63.

Similarly prepared from 2-fluorophenylglycine is 2-phthalimido-2-(2-fluorophenyl)acetic acid as a white solid: mp 174.5-180.5°C; 1H NMR (DMSO-d₆) δ 13.8 (br s, 1 H), 7.65-7.15 (m, 4H), 6.18 (s, 1 H); 13C NMR (DMSO-d₆) δ 168.1, 166.8, 162.1, 158.2, 135.0, 130.9, 130.8, 130.5, 130.4, 124.1. 123.6, 121.8, 121.6, 115.3, 114.9, 48.9. Anal. Calculated for C₁₆H₁₀NO₄F. Theoretical: C, 64.22; H, 3.37; N, 4.68. Found: C, 63.93; H, 3.27; N, 4.68.

### EXAMPLE 5

Similarly prepared according to the procedure of Referencial Example 3 from 2-phthalimido-2-(4-fluorophenyl)acetic acid, carbonyldiimidazole, 4-N,N-dimethylaminopyridine and concentrated ammonium hydroxide is 2-phthalimido-2-(4-fluorophenyl)acetamide which can be recrystallized from tetrahydrofuran to afford 0.76 g (51%) of the product as white crystals: mp 180-183°C; 1H NMR (DMSO-d₆) δ 8.00-7.55 (m, 4 H), 7.64 (s, 1 H), 7.60-7.40 (m, 3 H), 7.25-7.05 (m, 2 H), 5.83 (s, 1 H). Anal. Calculated for C₁₆H₁₁N₂O₃F. Theoretical: C, 64.43; H, 3.72; N, 9.39. Found: C, 64.16; H, 3.62; N, 9.18.

Likewise from 2-phthalimido-2-(2-fluorophenyl)acetic acid there is obtained 2-phthalimido-2-(2-fluorophenyl)acetamide as small white crystals: mp 197-201°C; 1H NMR (DMSO-d₆) δ 8.05-7.75 (m, 5 H), 7.65-7.05 (m, 5 H), 6.06 (s, 1 H), 13C NMR (DMSO-d₆) δ 167.4, 166.9, 162.2, 158.3, 134.6, 131.3, 131.2, 131.1, 130.2, 130.0, 123.9, 123.8, 123.2, 122.4, 115.1, 114.8, 49.9.

### REFERENTIAL EXAMPLE 6

To a stirred solution of 2-phthalimido-4-methylpentanoic acid (1.32 g, 5.0 mmol) in tetrahydrofuran (25 mL) are added carbonyldiimidazole (0.81 g, 5.0 mmol) and a few crystals of 4-N,N-dimethylaminopyridine followed by 15 mL of tetrahydrofuran. The reaction mixture is stirred at room temperature for 1 hour, then 1 mL of concentrated ammonium hydroxide is added. After 10 minutes, the reaction mixture is diluted with 50 mL water. The resulting slurry is partially concentrated to remove the tetrahydrofuran and filtered. The solid is washed with water and dried in vacuo (60°C, < 133Nm⁻² (< 1mm)) to afford 1.16 g (89%) of 2-phthalimido-4-methylpentanamide as a white powder: mp 173-176°C; 1H NMR (DMSO-d₆, 250 MHz) δ 7.95-7.79 (m, 4 H, Ar), 7.61 (br s, 1 H, CONH2), 7.22 (br s, 1 H, CONH2), 4.73-4.60 (m, 1 H), 2.30-2.10 (m, 1 H), 1.95-1.80 (m, 1H), 1.45-1.25 (m, 1H); 13C NMR (DMSO-d₆) δ: 170.4, 167.7, 134.4, 131.5, 123.1, 51.3, 36.4, 24.7, 23.2, 20.6. Anal. Calculated for C₁₄H₁₆N₂O₃. Theoretical: C, 64.60; H, 6.20; N, 10.76. Found: C, 64.63; H, 6.11; N, 10.70.

### EXAMPLE 7

To a stirred solution of histidine (3.17 g, 20.0 mmol) and sodium carbonate (2.23 g, 21 mmol) in 50 mL of water is added N-carboethoxyphthalimide (4.52 g, 20.0 mmol). After 1.5 hour, the reaction slurry is filtered. The filtrate is stirred and the pH adjusted to 1-2 with 4 N hydrochloric acid. The resulting slurry is filtered and the solid washed with water and dried in vacuo (60 C, < 133Nm⁻² (< 1mm)) to afford 3.65 g (64%) of 2-phthalimido-3-(imidazol-4-yl)propionic acid as a white powder: mp 280-285°C; 1H NMR (DMSO-d₆, 250 M Hz) δ 12.5 (br s, 1H), 7.90-7.60 (m, 6H), 6.80(s, 1H), 4.94 (t, 1H, J =_7.8), 3.36 (d, 2H, J = 7.8); 13C NMR (DMSO-d₆) δ 170.1, 167.1, 134.8, 134.6, 133.2, 131.1, 123.2, 116.3, 52.4, 25.8; Anal. Calculated for C₁₄H₁₁N₃O₄. Theoretical: C, 58.95; H, 3.89; N, 14.73. Found: C, 58.80; H, 3.88; N, 14.66.

### EXAMPLE 8

To a stirred mixture of 3-amino-3-(4-methoxyphenyl)propionic acid (1.95 g, 10.0 mmol) and sodium carbonate (1.11 g, 10.5 mmol) in 200 mL of acetonitrile-water 1:1 is added N-carboethoxyphthalimide (2.26 g, 10.0 mmol). After 1 hour, the reaction slurry is filtered. The filtrate is concentrated to remove the acetonitrile and the pH adjusted to 1-2 with 4 N hydrochloric acid and stirred over night. The resulting slurry is filtered and the solid washed with water. The solid is dried in vacuo (60 C, < 133Nm⁻² (< 1mm) to afford 2.82 g (87%) of the 3-phthalimido-3-(4-methoxyphenyl)propionic acid as a white powder: mp 160-164°C; 1H NMR (DMSO-d₆, 250 MHz) δ 12.5 (br s, 1H), 7.95-7.80 (m, 4 H), 7.36 (d, 2 H, J = 8.7), 6.92 (d, 2 H, J = 8.4 Hz), 5.18-5.10 (m, 1 H), 3.70-3.15 (m, 2 H); 13C NMR (DMSO-d₆) δ 171.7, 167.6, 158.6, 134.6, 131.0, 130.8, 128.3, 123.1, 113.9, 55.0, 49.6, 35.9. Anal. Calculated for C₁₈H₁₅NO₅. Theoretical: C, 66.46; H, 4.63; N, 4.31. Found: C, 66.25; H, 4.65; N, 4.28.

Similarly from 3-amino-3-(3-methoxyphenyl)propionic acid there is obtained 3-phthalimido-3-(3-methoxyphenyl)propionic acid as white crystals: mp 111-115°C; 1H NMR (DMSO-d₆, 250 MHz) δ 12.5 (br s, 1H), 7.94-7.81 (m, 4 H), 7.32-7.23 (m, 1H), 7.02-6.85 (m, 3 H), 5.70-5.60 (m, 1 H), 3.77-3.67 (s, 3H), 3.56-3.15 (m, 2 H); 13C NMR (DMSO-d₆) δ 171.6, 167.6, 159.2, 140.4, 134.7, 131.0, 129.7, 123.2, 119.0, 112.9, 112.7, 54.9, 50.0, 35.8.

Likewise from 3-amino-3-(2-methoxyphenyl)propionic acid there is obtained 3-phthalimido-3-(2-methoxyphenyl)propionic acid as a white powder: mp 163-168°C; 1H NMR (DMSO-d₆, 250 MHz) δ 12.5 (br s, 1H), 7.95-7.80 (m, 4 H), 7.45-6.90 (m, 4H), 6.05-5.92 (m, 1 H), 3.78 (s, 3H) 3.55-3.05 (m, 2 H); 13C NMR (DMSO-d₆) δ 171.7, 167.5, 156.1, 134.5, 131.0, 128.9, 127.3, 126.1, 123.0, 120.1, 111.0, 55.5, 45.3, 35.1.

### EXAMPLE 9

By following the procedure of Referencial Example 6 utilizing 3-phthalimido-3-(4-methoxyphenyl)propionic acid, there is obtained 3-phthalimido-3-(4-methoxyphenyl)propionamide as a white, powder: mp 183-288°C; 1H NMR (DMSO-d₆, 250 MHz) δ 7.90-7.75 (m, 4 H, Ar), 7.58 (br s, 1 H, CONH₂), 7.38 (d, 2H, J = 8.6), 6.91 (d, 3H, J = 8.6), 5.73 (t, 1H, J = 7.8), 3.23(d, 2H, J = 7.9); 13C NMR (DMSO-d₆) δ: 171.2, 167.6, 158.5, 134.5, 131.3, 131.2, 128.4, 123.0, 113.7, 55.0, 49.9, 36.8.. Anal. Calculated for C₁₈H₁₆N₂O₄. Theoretical: C, 66.66; H,4.97; N, 8.64. Found: C, 66.27; H, 5.04; N, 8.40.

### EXAMPLE 10

To a stirred mixture of 3-amino-3-(4-cyanophenyl)propionic acid (3.80 g, 20.0 mmol) and sodium carbonate (2.23 g, 21 mmol) in 100 mL of water is added N-carboethoxyphthalimide (4.52 g, 20.0 mmol). After 2 hour, the reaction slurry is filtered and the pH of the stirred filtrate adjusted to 1-2 with 4 N hydrochloric acid. The resulting gel is extracted with ethyl acetate (3x 30 mL). The extract is dried over magnesium sulfate and concentrated in vacuo. The crude product is recrystallized from 10% aqueous acetonitrile and then recrystallized from 20% aqueous methanol. The product is dried in vacuo (60°C, < 133Nm⁻² (< 1mm)) to afford 1.5 g (23%) of 3-phthalimido-3-(4-cyanophenyl)propionic acid as a white powder: mp 134-137°C; 1H NMR (DMSO-d₆, 250 MHz) δ 12.5 (br s, 1H), 7.95-7.56 (m, 8 H),.5.76 (t, 1 H, J = 7.7), 3.57-3.15 (m, 2 H); 13C NMR (DMSO-d₆) δ 171.5, 167.6, 144.2, 134.8, 132.6, 131.1, 128.1, 123.3, 118.5, 49.7, 35.5.

Likewise from 3-amino-3-(3-cyanophenyl)propionic acid there is obtained 3-phthalimido-3-(3-cyanophenyl)propionic acid as a white powder: mp 172-175°C; 1H NMR (DMSO-d₆, 250 MHz) δ 12.5 (br s, 1H), 8.05-7.51 (m, 8 H), 5.82-5.70 (m, 1 H), 3.63-3.20(m, 2 H); 13C NMR (DMSO-d₆) δ 171.5, 167.6, 140.3, 134.6 132.0, 131.5, 131.2, 130.7, 129.8, 123.22, 118.5, 111.6, 49.3, 35.6.

### EXAMPLE 11

By following the procedure of Referencial Example 6 utilizing 3-phthalimido-3-(4-cyanophenyl)propionic acid, there is obtained 3-phthalimido-3-(4-cyanophenyl)propionamide as a white powder: 1H NMR (DMSO-d₆, 250 MHz) δ 8.05-7.50 (m, 9 H), 6.97 (s, 1 H), 5.87-5.72 (m, 1 H), 3.44-3.12 (m, 2 H); 13C NMR (DMSO-d₆) δ 170.8, 167.6, 144.6, 134.6, 132.4, 131.1, 127.9, 123.2, 118.5, 110.3, 49.8, 36.4.

Similarly from 3-phthalimido-3-(3-cyanophenyl) propionic acid (1.60 g, 5.0 mmol), there is obtained 3-phthalimido-3-(3-cyanophenyl)propionamide as a white powder: mp 217-220°C; 1H NMR (DMSO-d₆, 250 MHz) δ 8.05-7.40 (m, 9 H), 6.99 (br s, 1 H), 5.90-5.75 (m, 1H), 3.50-3.10 (m, 2H); 13C NMR (DMSO-d₆) δ: 171.0, 167.7, 140.8, 134.6, 132.2, 131.5, 131.4, 130.8, 129.9, 123.2, 118.7,111.5, 49.7, 36.7.

### EXAMPLE 12

To a stirred solution of 3-amino-3-(4-methoxyphenyl)propionic acid methyl ester hydrochloride (1.50 g, 6.1 mmol) and sodium carbonate (90.65 g, 6.1 mmol) in 40 mL of water was added N-carboethoxyphthalimide (1.34 g, 6.1 mmol) in 12 mL of acetonitrile. The reaction mixture was stirred at room temperature for 1 hour. The reaction mixture was partially concentrated and this mixture was stirred for 72 hours. The resulting slurry was filtered and the solid was washed with copious amount of water. The solid was dried *in vacuo* (30°C, < 133Nm⁻² (< 1 mm)) to afford 1.70 g (50%) of methyl 3-phthalimido-3-(4-methoxyphenyl)propionate as a white powder. mp 65-66°C; 1H NMR (DMSO-d₆, 250 MHz) δ 7.83-7.91 (m, 4H), 6.88-7.3 (m, 4H), 5-80 (dd, 1H, J = 7.5, 2.5), 3.72 (S, 3H), 3.54 (3, 3H), 3.2-3.6 (m, 2H); 13C NMR (DMSO-d₆) δ 170.7, 167.5, 158.7, 134.6, 131.0, 130.5, 128.3, 123.2, 113.9, 55.0, 51.5, 49.4, 35.4. Anal. Calcd for C₁₉H₁₇NO₅: C, 67.25; H, 5.05; N, 4.13; Found: C, 66.96; H, 5.00; N, 4.11.

### EXAMPLE 13

To a stirred solution of 3-amino-3-(4-methoxyphenyl)propionic acid ethyl ester hydrochloride (1.00 g, 3.85 mmol) and sodium carbonate (0.41g, 3.85 mmol) in 40 mL of water was added N-carboethoxyphthalimide (0.84 g, 3.85 mmol) in 10 mL of acetonitrile. The reaction was complete in one hour by TLC. The reaction mixture was partially concentrated to remove the acetonitrile. To the resulting mixture was added 0.5 mL of Et20 and the mixture was stirred for 1 hour at room temperature. The resulting slurry was filtered and the solid was washed with copious amounts of water. The solid was air-dried overnight to afford 1.02 g (75%) of ethyl 3-phthalimido-3-(4-methoxyphenyl)propionate as a white gum: mp 32°C; ¹H NMR (DMSO-d₆, 250 MHz) δ 7.86 (m, 4H), 6.90-7.37 (M, 4H), 5.66 (dd, 1H J1 = 7.5, J2 = 2.5), 4.00 (d, 2H, J = 7.5), 3.3-3.6 (M, 2H), 1.04 (t, 3H, J = 7.5 Hz); ¹³C (DMSO-d₆) δ 170.1, 167.5, 158.7, 134.7, 131.0, 130.5, 128.3, 123.2, 113.88, 60.1, 55.0, 49.5, 35.7, 13.8. Anal. Calcd for C₂₀H₁₉NO₅: C, 67.98; H, 5.42; N, 3.90; Found C, 67.78; H, 5.30; N, 3.92.

### EXAMPLE 14

To a stirred solution of 3-amino-3-(4'-methoxyphenyl)propionic acid propyl ester hydrochloride (1.50 g, 5.52 mmol) and sodium carbonate (0.59g, 5.52 mmol) in 50 mL of water was added N-carboethoxy-phthalimide (1.21 g, 5.52 mmol) in 12 mL of acetonitrile. The reaction was complete in one hour. The acetonitrile was removed in vacuo and 5 mL of ether was added to the mixture, which was stirred overnight at room temperature. The resulting slurry was filtered and the solid was washed with 60 mL of water. The solid was dried in vacuo (24°C, < 133Nm⁻² < 1 mm) to afford 1.69g (83.2%) of propyl 3-phthalimido-3-(4-methoxyphenyl)propionate as a white powder: mp 51.2-52.8°C; ¹H NMR (DMSO-d₆ 250 MHz) δ 7.86 (m, 4H), 6.92-7.33 (m, 4H), 5.66 (dd, 1H, J1 = 7.5, 2.5 Hz), 3.90 (t, 2H, J = 5 Hz), 3.72 (S, 3H), 3.3-3.63 (m, 2H), 1.42 (hex, 2H, J = 7.5 Hz), 0.75 (t, 3H, J = 7.5 Hz); ¹³C (DMSO-d₆) δ 170.2, 167.5, 158.7, 134.7, 131.0, 130.5, 128.3, 123.2, 113.9, 65.6; 55.0, 49.5, 21.3, 9.98. Anal. Calcd for C₁₉H₁₇NO₅: = C, 68.65; H, 5.76; N, 3.81; Found = C, 68.42; H, 5.49; N, 3.76.

### REFERENTIAL EXAMPLE 15

A stirred mixture of phenylglycine (1.51 g, 10.0 mmol) and phthalic dicarboxaldehyde (1.34 g, 10.0 mmol) in 10 mL of acetic acid under nitrogen was heated to reflux for 10 minutes. The resulting mixture was allowed to cool overnight and the resulting slurry filtered to afford 1.56 g of crude product. The crude product was recrystallized from acetic acid to afford after drying *in vacuo* < 133 Nm⁻² ((< 1 mm), 60 C) 0.95 g (36%) of 2-(1'-Oxo-isoindoline)-2-phenylethanoic acid as a white powder: 1H NMR (DMSO-d₆, 250 MHz) 7.85-7.30(m, 9 H, Ar), 6.01 (s, 1 H, CH), 4.64 (d, J = 17.4 Hz, 1 H), 3.92 (d, J = 17.4 H, 1 H); 13C NMR (DMSO-d₆) 171.2, 167.4, 142.0, 134.6, 131.6, 131.3, 128.9, 128.7, 128.4, 127.9, 123.6, 122.9, 57.9, 47.6; Anal. Calcd for C₁₆H₁₃NO₃ 0.13 H₂O. Theory: C, 71.29; h, 4.95; N, 5.20. Found: C, 71.29; h, 4.86; N, 5.26.

### REFERENTIAL EXAMPLE 16

A mixture of 2-(1'-oxo-isoindoline)-2-phenylethanoic acid (0.50 g, 1.87 mmol) and carbonyl diimidazole (CDI, 0.319 g, 1.96 mmol) in 20 mL of tetrahydrofuran under nitrogen was stirred for 2.5 h, then 0.3 mL of 15 N ammonium hydroxide was added. The resulting mixture was stirred for 20 minutes, concentrated to an oil and diluted with 25 mL of water. The mixture was stirred and the resulting slurry filtered to afford after drying 0.38 g (76%) of 2-(1'-Oxo-isoindoline)-2-phenylacetamide as a white powder: ¹H NMR (DMSO-d₆, 250 MHz) 8.10-7.20 (m, 11 H), 6.03 (s, 1 H), 4.80 (d, J = 17.7 Hz, 1 H), 3.90 (d, J = 17.7 Hz, 1 H); ¹³C NMR (DMSO-d₆) 167.4, 142.2, 136.0, 131.5, 131.4, 128.7, 128.5, 128.0, 127.7, 123.4, 122.8, 57.5, 48.0; Anal. Calcd for C₁₆H₁₄N₂O₂: Theory C, 72.17; H, 5.30; N, 10.52. Found: C, 72.00; H, 5.27; N, 10.56.

### REFERENTIAL EXAMPLE 17

To a stirred solution of 3-phenyl-3-(1'-oxo-isoindoline)propanoic acid (0.703 g, 2.50 mmol) in 15 mL of tetrahydrofuran under nitrogen was added carbonyldiimidazole (0.438 g, 2.70 mmol), and a few crystals of 4-N,N-dimethylaminopyridine [DMAP]. The reaction mixture was stirred for 1.5 hours and then 0.25 mL of 15 N ammonium hydroxide was added. After 20 minutes, the reaction mixture was concentrated *in vacuo* and the residue slurried in water. The resulting solid was isolated by filtration and dried *in vacuo* to afford 0.58 g (80%) of crude product as an off-white powder. The crude product was recrystallized from ethanol to afford 0.403 g (57%) of 3-Phenyl-3-(1'-oxo-isoindoline)propanamide as white prisms: ¹H NMR (DMSO-d₆, 250 MHz) 7.8-7.2 (m, 10 H), 6.92 (br s, 1 H), 5.81 (overlapping dd, 1 H) 4.59 (d, J = 17.5 Hz, 1 H), 4.16 (d, J = 17.5 Hz, 1 H), 3.1-2.8 (m, 2 H); ¹³C NMR (DMSO-d₆) 171.3, 167.0, 140.7, 132.2, 131.4, 128.6, 127.9, 127.5, 126.9, 123.5, 122.8, 51.5, 46.3, 37.9; Anal. Calcd for C₁₇H₁₆N₂O₂: Theory C, 72.84; H, 5.75; N, 9.99. Found: C, 72.46; H, 5.68; N, 9.91.

### EXAMPLE 18

By following the procedure of Referencial Example 15 utilizing 3-amino-3-(4'-methoxyphenyl)propanoic acid to afford 1.52 g of crude product as an off white solid from the reaction mixture. The filtrate was concentrated and the residue slurried in 25 mL of ethyl acetate to afford after filtration an additional 1.27 g (41%) of crude product as a pale green powder. The combined crude products were recrystallized from 280 mL of ethyl acetate to afford after drying 1.69 g (55%) of 3-(4'-Methoxyphenyl)-3-(1'-oxo-isoindoline)propanoic acid as an off-white solid: Anal. Calcd for C₁₈H₁₇NO₄: Theory C, 69.44; H, 5.50; N, 4.50. Found: C, 69.33; H, 5.45; N, 4.49.

### EXAMPLE 19

By following the procedure of Referencial Example 17 utilizing 3-(4'-methoxyphenyl)-3-(1'-oxo-isoindoline)propanoic acid to afford 0.49 g (82%) of crude product. The crude product was recrystallized from ethyl acetate (40 mL) to afford 0.27 g (45%) of 3-(4'-Methoxyphenyl)-3-(1'-oxo-isoindoline)propionamide as white needles: ¹H NMR (DMSO-d₆, 250 MHz) 7.8-7.4 (m, 5 H), 7.29 (d, 2 H, J = 9 Hz), 6.91 (d, 2 H, J = 9 Hz), 5.78 (t, 1 H, J = 8 Hz), 4.55 (d, 1 H, J = 17.5 Hz), 4.11 (d, J = 17.5 Hz, 1 H), 3.72 (s, 3 H), 3.05-2.75 (m, 2 H); ¹³C NMR (DMSO-d₆) 171.2, 166.8, 158.4, 141.6, 132.2,131.8, 131.2, 128.1, 127.8, 123.3, 122.7, 113.8, 55.0, 51.0, 46.1; Anal. Calcd for C18H18N203-0.38 H₂O: Theory C, 68.58; H, 5.99; N, 8.80. Found: C, 68.58; H, 5.86; N, 8.80.

### EXAMPLE 20

By following the procedure of Referencial Example 15 utilizing 3-amino-3-(3',4'-dimethoxyphenyl)propanoic acid with the following exceptions. The reaction mixture (solution) was concentrated to a thick oil which was diluted with 10 mL of ethyl acetate. The resulting slurry was filtered, the solid washed with ethyl acetate and then dried *in vacuo* >133 Nm⁻² ((> 1 mm), 60 C) to afford 2.77 g (81%) of 3-(3',4'-Dimethoxyphenyl)-3-(1'-oxo-isoindoline)propanoic acid as a white powder: mp 146.5-148.5 C; ¹H NMR (DMSO-d₆, 250 MHz) 12.34 (br s, 1 H, CO2H), 7.8-7.4 (m, 4 H), 7.1-6.8 (m, 3 H), 5.85-5.65 (m, 1 H), 4.51 (d, 1 H, J = 18 Hz), 4.13 (d, 1 H, J = 18 Hz), 3.75 (s, 3 H), 3.73 (s, 3 H), 3.3-3.0 (m, 2 H); ¹³C NMR (DMSO-d₆) 171.8, 166.7, 148.7, 148.3, 141.6, 132.1, 131.6, 131.3, 127.8, 123.4, 122.7, 119.2, 111.7, 111.2, 55.5, 55.4, 46.3, 36.8 ; Anal. Calcd for C₁₉H₁₉NO₅: Theory C, 66.85; H, 5.61; N, 4.10. Found: C, 67.19; H, 5.57; N, 3.73.

### EXAMPLE 21

By following the procedure of Referencial Example 17 utilizing 3-(3',4'-dimethoxyphenyl)-3-(1'-oxo-isoindoline)propanoic acid with the following changes. The crude product did not precipitate from water immediately. The product crystallized from aqueous solution upon sitting for several days after an ether wash to afford 0.26 g (22%) of 3-(3',4'-Dimethoxyphenyl)-3-(1'-oxo-isoindoline)propionamide as white needles: ¹H NMR (DMSO-d₆, 250 MHz) 7.8-7.4 (m, 5H), 7.1-6.85 (m, 4 H), 5.76 (m, 1 H), 4.57 (d, 17.6 Hz, 1 H), 4.15 (d, J = 17.6 Hz, 1 H), 3.74 (s, 3 H), 3.72 (s, 3 H), 3.1-2.8 (m, 2 H); ¹³C NMR (DMSO-d₆) 171.2, 166.8, 148.6, 148.1, 141.6, 132.2, 132.2, 131.2, 127.8, 123.4, 122.7, 119.0, 111.6, 111.0, 55.4, 51.4, 46.2, 37.9; Anal. Calcd for C₁₉H₂₀N₂O₄: Theory C, 67.05; H, 5.92; N, 8.23. Found: C, 66.74; H, 5.88; N, 8.02.

### EXAMPLE 22

To a stirred solution of 3-amino-3-(3',4'-diethoxyphenyl)propionic acid (1.03 g, 4.07 mmol) and sodium carbonate (0.453 g, 4.27 mmol) in a 1/1 mixture of 150 mL of acetonitrile/water (heated to 45 C to dissolve) was added N-carbethoxyphthalimide (0.89 g, 4.07 mmol). The reaction mixture was stirred 1h, then partially concentrated *in vacuo* to remove the acetonitrile to afford a pale yellow solution. The stirred solution was acidified to pH 0-1 with 4 N hydrochloric acid to form a gum. The mixture was stirred overnight. The gum had not solidified, 1 mL of ether was added to mixture. The gum solidified on stirring, the slurry was filtered and the solid dried to afford 1.94 g (94%) of 3-(3',4'-diethoxyphenyl)-3-(phthalimido)propionic acid as a yellow solid: ¹H NMR (DMSO-d₆, 250 MHz) 12.41 (br s, 1 H, COOH), 8.10-7.75 (m, 4 H, Ar), 7.15-6.85 (m, 3 H, Ar), 5.62 (overlapping dd, 1 H), 4.20-3.90 (m, 4 H, 2 OCH2), 3.51 (dd, 1 H, J = 16.5, 9 Hz), 3.25 (dd, 1 H, J = 16.5, 7 Hz), 1.5-0.9 (m, 6 H, 2 CH3) ¹³C NMR (DMSO-d₆) 1717, 167.6, 147.9, 147.8, 134.6, 131.3, 131.0, 123.1, 119.4, 113.2, 112.7, 63.8, 63.7, 50.0, 36.0, 14.6, 14.6; Anal. Calcd for C₂₁H₂₁NO₆: Theory C, 65.79; H, 5.52; N, 3.65. Found: C, 65.54; H, 5.55; N, 3.62.

### EXAMPLE 23

The procedure of Referential Example 17 was followed with the following changes. The reaction mixture concentrated *in vacuo* and to an oil which was diluted with water (20 mL) and ether (1 mL) and the mixture stirred overnight. The resulting slurry was filtered and the solid dried *in vacuo* to afford 0.41 g (41%) of crude product. The crude product was recrystallized from ethyl acetate to afford 0.265 g (27%) of 3-(3',4'-diethoxyphenyl)-3-phthalimidopropionamide as white crystals: ¹H NMR (DMSO-d₆, 250 MHz) 8.00-7.60 (m, 4 H, Ar), 7.55 (br s, 1 H, NH), 7.03 (br s, 1 H, NH), 6.89 (br s, 3 H, Ar), 5.66 (t, 1 H, J = 8 Hz), 4.15-3.85 (m, 4 H), 3.3-3.05 (m, 2 H), 1.5-1.15 (m, 6 H); ¹³C NMR (DMSO-d₆) 171.2, 167.6, 147.8, 147.6, 134.5, 131.6, 131.2, 123.0, 119.5, 113.0, 112.7, 63.7, 63.6, 50.2, 36.9, 14.6, 14.6; Anal. Calcd for C₂₁H₂₂N₂O₅: Theory C, 65.96 H, 5.80; N, 7.33. Found: C, 65.45; H, 5.55; N, 7.20.

### EXAMPLE 24

To a stirred solution of sodium carbonate (1.45 g, 13.7 mmol) in 500 mL of water-acetonitrile (1:1, v/v) was added 3-amino-3-(4'-propoxyphenyl)propionic acid (3.05 g, 13.7 mmol) as a solid. The mixture was warmed to 30-40°C to dissolve the solids. The reaction mixture was allowed to cool to room temperature and N-carboethoxyphthalimide (3.00 g, 13.7 mmol) was added and the reaction mixture was stirred for one hour at room temperature. The reaction mixture was then partially concentrated to remove the acetonitrile and the pH of the resulting solution was adjusted to approximately 3 with 4N hydrochloric acid. The resulting slurry was stirred overnight and then filtered and the solid was washed with copious amounts of water. The solid was dried *in vacuo* (60°C, < 133Nm⁻² (<1mm)) to afford 3.64 g (75%) of 3-phthalimido-3-(4'-propoxyphenyl)propionic acid as a white powder: mp 142.5-143.6°C, ¹H NMR(DMSO-d₆, 250 MHz) δ 12.43 (br s, 1 H), 7.80-7.95 (m, 4 H), 7.34 (d, 2 H, J = 9 Hz), 6.89 (d, 2 H, J = 9 Hz), 5.63 (overlapping dd, 1 H), 3.88 (t, 2 H, J = 7 Hz), 3.45 (dd, 1 H, J1 = 9 Hz, J2 = 16.5 Hz), 3.30 (dd, 1 H, J1 = 7 Hz, J2 = 16.5 Hz), 1.60-1.85 (m, 2 H), 0.95 (t, 3 H, J = 7 Hz); ¹³C (DMSO-d₆) δ 171.8, 167.6, 158.6, 134.7, 131.1, 130.8, 128.3, 123.2, 114.4, 68.9, 49.7, 36.0, 22.0, 10.3; Anal. Calcd. for C₂₀H₁₉NO₅. Theoretical: C, 67.98; H, 5.42; N, 3.96. Found: C, 67.90; H, 5.40; N, 4.00.

### EXAMPLE 25

To a stirred solution of 3-phthalimido-3(4'-propoxyphenyl)propionic acid (1.41 g, 4.0 mmol) in 25 mL of tetrahydrofuran under nitrogen was added carbonyldiimidazole (0.68 g, 4.2 mmol) followed by a catalytic amount of dimethylaminopyridine. The mixture was stirred for 45 minutes at room temperature. To the reaction mixture was then added concentrated ammonium hydroxide (0.29 mL, 4.4 mmol) and the reaction mixture was stirred for 30 minutes at room temperature. The mixture was then diluted with 10 mL of water and the tetrahydrofuran was removed *in vacuo*. The resulting slurry was filtered and the solid was washed with copious amounts of water. The solid was dried *in vacuo* (60°C, < 133Nm⁻² (< 1mm)) to afford 1.2 g of crude product. The crude product was purified by dissolving in 200 mL of ethyl acetate, stirring for 3h and then concentrating to an 80 mL volume. The resulting slurry was filtered and the solid was washed with ethyl acetate (2x20 mL). The solid was air-dried to afford 0.513 g (36%) of 3-phthalimido-3-(4'-propoxyphenyl)propionamide as a white powder: mp 109.5-110.4°C; ¹H NMR (DMSO-d₆, 250 MHz) δ 7.85 (br s, 4 H), 7.55 (br s, 1 H), 7.33 (d, 2 H, J = 8 Hz), 6.75-7.00 (m, 3 H), 5.69 (t, 1 H, J = 8 Hz), 3.88 (t, 2 H, J = 6 Hz), 3.10-3.30 (m, 2 H), 1.60-1.80 (m, 2 H), 0.95 (t, 3 H, J = 7 Hz); ¹³C NMR (DMSO-d₆) δ 171.2, 167.7, 158.0, 134.5, 131.23, 131.19, 128.4, 123.1, 114.3, 68.9, 49.9, 36.9, 22.0, 20.4, 10.4; Anal. Calcd. for C₂₀H₂₀N₂O₄ @0.37 H₂O. Theoretical: C, 66.90; H, 5.61; N, 7.80. Found: C, 66.90; H, 5.52; N, 7.75.

### EXAMPLE 26

To 40 mL of stirred ethanol at 0°C under nitrogen was slowly added thionyl chloride (3.3 mL, 45 mmol) followed by addition of 3-amino-3-(3'-pyridyl)propionic acid (2.65 g, 15 mmol). The reaction mixture was allowed to slowly warm to room temperature and then refluxed for 3 hours. After 2 hours at reflux all of the solid had dissolved. The reaction mixture was allowed to cool to room temperature and stirred overnight. The slurry was filtered and the solid was washed with copious amounts of ethanol. The solid was dried *in vacuo* (60°C, < 133Nm⁻² (<1mm)) to afford 3.17 g (79%) of ethyl 3-amino-3-(3'-pyridyl)propionate hydrochloride as a white powder: 1H NMR (DMSO-d₆, 250 MHz) δ 9.32 (br s, 3 H), 9.21 (br s, 1 H), 8.87-8.95 (m, 2 H), 8.09-8.14 (m, 1 H), 4.93 (br s, 1 H), 3.90-4.15 (m, 2 H), 3.20-3.38 (m, 2 H), 1.11 (t, 3 H, J = 7 Hz); 13C NMR (DMSO-d₆) δ 168.8, 144.5, 142.8, 142.6, 136.2, 126.7, 60.7, 47.9, 37.2, 13.9.

### EXAMPLE 27

By following the procedure of Example 13 utilizing Prepared as described earlier for ethyl 3-phthalimido-3-(4'-methoxyphenyl)propionate from ethyl 3-amino-3-(3'-pyridyl)propionate hydrochloride. Ethyl 3-phthalimido-3-(3'-pyridyl)propionate was isolated as a white powder (0.43 g, 71%): mp 72.3-72.8°C; ¹H NMR (DMSO-d₆, 250 MHz) δ 8.45-8.70 (m, 2 H), 7.80-8.00 (m, 5 H), 7.35-7.45 (m, 1 H), 5.78 (dd, 1 H, J1= 6.5 Hz, J2 = 9.5 Hz), 4.01 (q, 2 H, J = 7 Hz), 3.62 (dd, 1 H, J1= 6.5 Hz, J2 = 16.4 Hz), 3.41 (dd, 1 H, J1= 9.5 Hz, J2 = 16.4 Hz), 1.05 (t, 3 H, J = 7 Hz); ¹³C NMR (DMSO-d₆) δ 169.9, 167.5, 148.96, 148.4, 134.9, 134.7, 134.0, 131.0, 123,6, 123.3, 60.3, 47.9, 35.2, 13.8; Anal. Calcd. for C18H16N2O4. Theoretical: C, 66.66; H, 4.97; N, 8.64. Found: C, 66.51; H, 4.94; N, 8.56.

### EXAMPLE 28

By following the procedure of Example 24 utilizing 3-amino-3-(3',4'-dimethoxyphenyl)propionic acid. 3-Phthalimido-3-(3',4'-dimethoxyphenyl)propionic acid was isolated as a white powder (5.30 g, 75%): ¹H NMR (DMSO-d₆, 250 MHz) δ 12.44 (br s,1 H), 7.70-8.00 (m, 4 H), 6.85-7.10 (m, 3 H), 5.63 (dd, 1 H, J1 = 7 Hz, J2 = 9 Hz), 3.74 (s, 3 H), 3.73 (s, 3 H), 3.53 (dd, 1 H, J1 = 9 Hz, J2 = 16.5 Hz), 3.26 (dd, 1 H, J1 = 7 Hz, J2 = 16.5 Hz), ¹³C NMR (DMSO-d₆) δ 171.8, 167.7, 148.6, 148.4, 134.7, 131.3, 131.1, 123.2, 119.3, 111.7, 111.0, 55.48, 55.46, 50.1, 36.1.

### EXAMPLE 29

By following the procedure of Example 25 utilizing 3-phthalimido-3-(3,4'-dimethoxyphenyl)propionic acid, 3-Phthalimido-3-(3',4'-dimethoxyphenyl)propionamide was isolated as a white powder (0.314 g, 52%): mp 188.8-190.0°C; ¹H NMR (DMSO-d₆, 250 MHz) δ 7.7-8.0 (m, 4 H), 7.54 (br s, 1 H), 6.7-7.1 (m, 4 H), 5.67 (t, 1 H, J = 8 Hz), 3.73 (s, 3 H), 3.72(s, 3 H), 3.20 (d, 2 H, J = 8 Hz); ¹³C NMR (DMSO-d₆) δ 171.2, 167.7, 148.5, 134.7, 134.5, 131.7, 131.2; 123.1, 119.4, 111.6, 111.2, 55.5, 50.3, 37.0; Anal. Calcd. for C₁₉H₁₈N₂O₅. Theoretical: C, 64.40; H, 5.12; N, 7.91. Found: C, 64.01; H, 5.14; N, 7.64.

### EXAMPLE 30

The procedure of Example 26 was followed except the reaction was run at room temperature. Ethyl 3-Amino-3-(3',4'-dimethoxyphenyl)propionate was isolated as a white powder (2.04 g, 88%): 1H NMR (DMSO-d₆, 250 MHz) δ 8.75 (br s, 3 H), 7.30-7.35 (m, 1 H), 6.90-7.05 (m, 2 H), 4.50 (dd, 1 H, J1 = 6 Hz, J2 = 9 Hz), 3.90-4.10 (m, 2 H), 3.77 (s, 3 H), 3.75 (s, 3 H), 3.19 (dd, 1 H, J1 = 6 Hz, J2 = 16 Hz), 2.98 (dd, 1 H, J1 = 9 Hz, J2 = 16 Hz), 1.10 (t, 3 H, J = 7 Hz); 13C NMR (DMSO-d₆) δ 169.1, 149.0, 148.6, 128.9, 120.1, 111.4, 60.4, 55.6, 55.5, 50.9, 38.7, 13.9.

### EXAMPLE 31

By following the procedure of Example 13 utilizing ethyl 3-amino-3-(3',4'-dimethoxyphenyl)propionate. The reaction mixture was concentrated and the residue was dissolved in 20 mL of ethyl acetate and washed with water (3 x 20 mL). The organic phase was dried over sodium sulfate and then concentrated to afford 0.31 g (40%) of ethyl 3-phthalimido-3-(3',4'-dimethoxyphenyl)propionate as a yellow oil: 1H NMR (DMSO-d₆, 250 MHz) δ 7.80-7.95 (m, 4 H), 7.04 (s, 1 H), 6.85-6.98 (m, 2 H), 5.65 (dd, 1 H, J1 = 6 Hz, J2 = 10 Hz), 4.00 (q, 2 H, J = 7 Hz), 3.74 (s, 3 H), 3.73 (s, 3 H), 3.60 (dd, 1 H, J1 = 10 Hz, J2 = 16 Hz), 3.32 (dd, 1 H, J1 = 6 Hz, J2 = 16 Hz), 1.05 (t, 3 H, J = 7 Hz); 13C NMR (DMSO-d₆) δ 170.2, 167.5, 148.58, 148.54, 134.7, 131.1, 130.9, 123.2, 119.2, 111.6, 111.0, 60.1, 55.5, 50.0, 35.9, 13.9; Anal. Calcd. for C₂₁H₂₁NO₆. Theoretical: C, 65.79; H, 5.52; N, 3.65. Found: C, 65.13; H, 5.73; N, 3.61.

### EXAMPLE 32

By following the procedure of Example 25 utilizing 3-phthalimido-3-(3,4'-dimethoxyphenyl)propionic acid and amylamine (1.0 equiv) to afford 2.15 g (84%) of crude product. The crude product was dissolved in 150 mL of ethyl acetate and then 50 mL of ether was added and the mixture stirred for 1 hour. The resulting slurry was filtered and the solid dried *in vacuo* to afford 1.28 g (50%) yield of 3-Phthalimido-3-(3',4'-dimethoxyphenyl)propionic amylamide as a white powder: mp 140.5-142.1°C; 1H NMR (DMSO-d₆, 250 MHz), d 8.05 (t, 1 H, J = 5 Hz), 7.85 (m, 4 H), 7.03 (br s, 1 H), 6.90 (m, 3 H), 5.68 (t, 1 H, J = 8 Hz), 3.73 (s, 3 H), 3.71 (s, 3 H), 3.19 (d, 2 H, J = 8 Hz), 2.8-3.1 (m, 2 H), 0.9-1.3 (m, 6 H), 0.74 (m, 3 H); 13C NMR (DMSO-d₆) δ 168.8, 167.7, 148.5, 148.3, 134.5, 131.5, 131.2, 123.1, 119.5, 111.6, 111.1, 55.4, 50.6, 38.2, 37.4, 28.7, 28.3, 21.7, 13.7; Anal. Calcd. for C₂₄H₂₈N₂O₅. Theoretical: C, 67.9; H, 6.65; N, 6.60. Found: C, 67.84; H, 6.70; N, 6.57.

### EXAMPLE 33

By following the procedure of Example 25 utilizing 3-phthalimido-3-(3,4'-dimethoxyphenyl)propionic acid and benzylamine (1.0 equiv) to afford 2.45 g (92%) of 3-Phthalimido-3-(3',4'-dimethoxyphenyl)propionic benzylamide as white powder: mp 208.4-209.8°C; 1H NMR (DMSO-d₆, 250 MHz) δ 8.60 (t, 1 H, J = 6 Hz), 7.78-7.92 (m, 4 H), 6.85-7.20 (m, 8 H), 5.73 (t, 1 H, J = 8 Hz), 4.10-4.30 (m, 2 H), 3.73 (s, 3 H), 3.72 (s, 3 H), 3.20-3.45 (m, 2 H); 13C NMR (DMSO-d₆) δ 169.1, 167.7, 148.5, 148.3, 139.2, 134.5, 131.4, 131.2, 127.9, 126.7, 123.1, 119.5, 111.5, 111.2, 101.9, 55.4, 55.37, 50.6, 41.7, 37.4; Anal. Calcd. for C₂₆H₂₄N₂O₅. Theoretical: C, 70.26; H, 5.44; N, 6.30. Found: C, 70.12; H, 5.53; N, 6.25.

### EXAMPLE 34

By following the procedure of Example 25 utilizing 3-phthalimido-3-(3,4'-dimethoxyphenyl)propionic acid and ethylamine (1.0 equiv). After the reaction mixture was concentrated, the residue was diluted with 20 mL of water and 1 mL of ether. The mixture was stirred for 1 hour to afford a slurry. The slurry was filtered and the solid dried in vacuo to afford 0.66 g (77%) of 3-Phthalimido-3-(3',4'-dimethoxyphenyl)propionic ethylamide compound as a white powder: mp 131.0-132.5°C; 1H NMR (DMSO-d₆, 250 MHz) δ 8.08 (t, 1 H, J = 5 Hz), 7.78-7.95 (m, 4 H), 7.03 (s, 1 H), 6.85-7.00 (m, 2 H), 5.69 (t, 1 H, J = 8 Hz), 3.74 (s, 3 H), 3.72 (s, 3 H), 3.18 (d, 2 H, J = 8 Hz), 2.98 (m, 2 H), 0.88 (t, 3 H, J = 7 Hz); 13C NMR (DMSO-d₆) δ 168.8, 167.7, 148.5, 148.2, 134.5, 131.6, 131.2, 123.1, 119.4, 111.6, 111.1, 55.5, 50.5, 37.3, 33.2, 14.5; Anal. Calcd. for C₂₁H₂₂N₂O₅. Theoretical: C, 65.96; H, 5.80; N, 7.33. Found: C, 65.85; H, 5.84; N, 7.24.

### EXAMPLE 35

By following the procedure of Example 24 utilizing 3-amino-3-(4'-ethoxyphenyl)propanoic acid. 3-Phthalimido-3-(4'-ethoxyphenyl)propionic acid was isolated as a white powder (2.52 g, 74%): mp 169.2-171.1°C; ¹H NMR (DMSO-d₆, 250 MHz) δ 7.75-8.00 (m, 4 H), 7.34 (d, 2 H, J = 8.7 Hz), 6.89 (d, 2 H, J = 8.7 Hz), 5.64 (overlapping dd, 1 H), 3.98 (q, 2 H, J = 7 Hz), 3.48 (dd, 1 H, J1 = 9 Hz, J2 = 16.5 Hz), 3.26 (dd, 1 H, J1 = 7 Hz, J2 = 16.5 Hz), 1.30 (t, 3 H, J = 7 Hz); ¹³C NMR (DMSO-d₆) δ 171.8, 167.7, 158.0, 134.7, 131.1, 130.8, 128.4, 123.2, 114.4, 63.0, 49.7, 36.1, 14.6; Anal. Calcd. for C19H17NO5. Theoretical: C, 67.25; H, 5.05; N, 4.13. Found: C, 67.05, H, 4.93; N, 4.17.

### EXAMPLE 36

By following the procedure of Example 25 utilizing 3-phthalimido-3-(4'-ethoxyphenyl)propionic acid to afford 1.3 g (88%) of crude product. Recrystallization of the crude material from ethyl acetate afforded 0.28 g (20%) of 3-phthalimdo-3-(4'-ethoxyphenyl)propionamide as a white powder: mp 190.6-191.2°C; ¹H NMR (DMSO-d₆, 250 MHz) δ 7.75-7.95 (m, 4 H), 7.54 (br s, 1 H), 7.33 (d, 2 H, J = 8.6 Hz), 6.75-6.98 (m, 3 H), 5.69 (t, 1 H, J = 8 Hz), 3.98 (q, 2 H, J = 7.Hz), 3.19 (d, 2 H, J = 8 Hz), 1.30 (t, 3 H, J = 7 Hz); ¹³C NMR (DMSO-d₆, 250 Mhz) δ 167.6, 154.1, 154.2, 130.9, 127.6, 124.7, 119.5, 110.6, 59.4, 46.3, 33.3, 11.0; Anal. Calcd. for C₁₉H₁₈N₂O₄ -0.37 H₂O. Theoretical: C, 66.14; H, 5.26; N, 8.12. Found: C, 66.14; H, 5.26; N, 7.81.

### EXAMPLE 37

A stirred mixture of 4-nitrophthalic acid anhydride (1.93 g, 10.0 mmol) and 3-amino-3-(4'-methoxyphenyl)propionic acid (1.95 g, 10.0 mmol) in 20 mL of acetic acid under nitrogen was heated to reflux for 4.5 hours. The reaction mixture was concentrated to an oil which was stirred in 18 mL of ethyl acetate overnight. The resulting slurry was filtered and the solid air-dried and then dried in vacuo (70°C, < 266Nm⁻² (< 2mm), 2 h) to afford 2.52 g (68%) of the product as a pale yellow powder contaminated with acetic acid and ethyl acetate. The material was dried in vacuo overnight at 90°C to afford a yellow glass which was slurried in 15 mL of ethyl acetate to afford after filtration and drying 1.72 g (46%) of 3-(4'-nitrophthalimido)-3-(4'-methoxyphenyl)propionic acid as a pale yellow powder contaminated with ethyl acetate: mp 90-91.5°C; ¹H NMR (DMSO-d₆) δ 8.75-8.60 (m, 1 H), 8.5 (m, 2 H), 8.12 (d, J = 8 Hz, 1 H), 7.38 (d, 2 H, J = 8.7 Hz H, Ar), 6.90 (d, 2 H, J = 8.7 Hz, Ar), 5.75-5.6 (m, 1 H, CHCO), 3.72 (s, 3 H, OMe), 3.47 (dd, 1 H, J = 8, 16.6 Hz), 3.33 (dd, 1 h, J = 7 Hz, 16.6 Hz); ¹³C NMR (DMSO-d₆) δ 171.6, 165.9, 165.7, 158.8, 151.5, 135.6, 132.4, 130.3, 129.8, 128.5, 124.7, 118.0, 113.9, 55.0, 50.2, 35.8.. Anal. Calcd for C₁₈H₁₄N₂O₇-1/3 EtOAc. Theory: C, 58.09 ; H, 4.20; N, 7.01. Found: C, 57.89; H, 4.29; N, 6.83.

### EXAMPLE 38

The procedure of Example 22 was followed utilizing 3-amino-3-(2'-napthyl)propionic acid and N-carbethoxyphthalimide to afford 1.43 g (83%) of crude product as an off-white powder. The crude product was purified by flash chromatography (silica gel, 4-4.5% methanol/methylene chloride) to afford 1.11 g of product as a white foam. The foam was slurried in 15 mL of ethanol to afford 1.03 g of 3-phthalimido-3-(2'-napthyl)propionic acid as a white powder contaminated with ethanol and methylene chloride: ¹H NMR (DMSO-d₆) δ; 12.56 (br s, 1H), 8.1-7.75 (m, 8H), 7.7-7.45 (m, 3 H), 5.89 (m, 1 H), 3.62 (dd, 1 H, J = 16.6, 9 Hz), 3.46 (dd, J = 16.6, 6.8 Hz); ¹³C NMR (DMSO-d₆) δ 171.8, 167.7, 136.3, 134.6, 132.6, 132.2, 131.1, 128.3, 127.9, 127.3, 126.3, 126.2, 125.6, 125.1, 123.2, 50.2, 35.8.

### EXAMPLE 39

The procedure of Referential Example 16 utilizing methyl 3-phthalimido-3-(2'-napthyl)propionate and carbonyldiimidazole to afford the crude product as a white powder. 3-Phthalimido-3-(2'-napthyl)propionamide was recrystallized from 40 mL of ethyl acetate to afford 0.259 g (35%) of the product as fine white prisms: ¹H NMR (DMSO-d₆) δ 8.15-7.75 (m, 8 H, Ar), 7.75-7.4 (m, 4 h, Ar and CONH), 6.94 (br s, 1 H, CONH), 5.93 (overlapping dd, 1 H, CHN), 3.55-3.15 (m, 2 H, CH₂CO); ¹³C NMR (DMSO-d₆) δ 171.2, 167.7, 136.7,134.5, 132.6, 132.2, 131.2, 128.1, 127.8, 127.3, 126.3, 126.1, 125.5, 125.2, 123.1, 50.4, 36.7. Anal. Calcd for C₂₁H₁₆N₂O₃. Theory: C, 73.24 ; H, 4.68; N, 8.13. Found: C, 73.07; H, 4.61; N, 7.91.

### EXAMPLE 40

A stirred suspension of 3-amino-3-(3',4'-dimethoxyphenyl)propionic acid hydrochloride (0.689 g, 2.50 mmol) and 4-pyridyldicarboxylic acid anhydride (0.373 g, 2.50 mmol) in 20 mL of acetic acid was refluxed for overnight. The cooled reaction was filtered to remove a trace amount of solid and the filtrate concentrated to a thick yellow oil. The oil was diluted with 20 mL of ethyl acetate and heated to reflux and allowed to cool to room temperature. The resulting slurry was filtered and the filtrate concentrated to afford a yellow oil which was purified by flash chromatography (silica gel, 2/8 ethyl acetate/methylene chloride) to afford 0.592 g (64%) of methyl 3-(1,3-dioxo-5-azaisoindol-2-yl)-3-(3',4'-dimethoxyphenyl)-propionate as a yellow oil which slowly solidified to afford a very pale yellow solid: ¹H NMR (DMSO-d₆) δ 8.15-7.75 (m, 8 H, Ar), 7.75-7.4 (m, 4 h, Ar and CONH), 9.13 (s, 1 H, Ar), 9.11 (d, 1 H, J = 4.8 Hz), 7.90 (d, 1 H, J = 4.8 Hz), 7.03 (s, 1 H), 6.93 (m, 2 H), 5.67 (overlapping dd, 1 H), 3.74 (s, 3 H), 3.73 (s, 3 H), 3.56 (s, 3 H), 3.65-3.30 (m, 2 H); ¹³C NMR (DMSO-d₆) δ 170.7, 166.9, 166.5, 156.0, 148.6, 148.5, 144.1, 138.7, 130.4, 125.2, 119.1, 116.9, 111.6, 111.1, 55.4, 51.6, 50.1,35.4..

### EXAMPLE 41

To a stirred solution of 3-amino-3-(4'-butoxy-3'-methoxyphenyl)propionic acid (1.31 g, 4.98 mmol) and sodium carbonate (0.554 g, 5.23 mmol) in a mixture of 100 mL of water and 100 mL of acetonitrile (mixture was warmed gently to dissolve solid, a small amount of brown solid which did not dissolve was removed by filtration) was added N-carbethoxyphthalimide (1.09 g, 4.98 mmol). The mixture was stirred for 1 hour, then partially concentrated *in vacuo* to remove the acetonitrile. The pH was adjusted to 0-1 with 4 N Hydrochloric acid. An oil formed, 3 mL of ether was added and the mixture stirred overnight. The oil did not solidify and was extracted into methylene chloride. The organic layer was dried (sodium sulfate) and concentrated to a yellow oil which was purified by flash chromatography (silica gel, 5/95 methanol/methylene chloride) to afford 1.02 g of 3-phthalimido-3-(4'-butoxy-3'-methoxyphenyl)propionic acid containing an unidentified impurity as a yellow oil which slowly crystallized: ¹H NMR (DMSO-d₆) δ 7.95-7.8 (m, 4 H, Ar), 7.03 (s, 1 H), 6.9 (m, 2 H), 5.61 (dd, 1 H, J = 9, 6.7 Hz), 3.91 (t, 2 H, J = 6.4 Hz), 3.74 (s, 3 H), 3.47 (dd, 1 H, J = 16.5, 6.7 Hz), 3.27 (dd, 1 H, J = 16.5, 6.7 Hz), 1.75-1.55 (m, 2 H) 1.5-1.3 (m, 2 H), 0.91 (t, 3 H, J = 7.3 Hz); ¹³C NMR (DMSO-d₆) δ 171.8, 167.7, 148.8, 147.8, 134.6, 131.3, 131.1, 123.2, 119.3, 112.9, 111.4, 67.8, 55.5, 50.1, 30.8, 18.7, 13.6.

### EXAMPLE 42

By following the procedure of Example 39 utilizing 3-phthalimido-3-(4'-butoxy-3'-methoxyphenyl)propionic acid and carbonyl diimidazole to afford 0.742 g (74%) of crude product as a pale yellow powder. The crude product was recrystallized from ethyl acetate (16 mL) to afford 0.517 (52%) of 3-phthalimido-3-(4'-butoxy-3'-methoxyphenyl)propionamide as fine fluffy white needles: HPLC 99.1%; ¹H NMR (DMSO-d₆) δ 7.95-7.75 (m, 4 H, Ar), 7.54 (br s, 1 H, CONH), 7.04 (s, 1 H, Ar), 7.0-6.75 (m, 2 H), 6.86 (br s, 1 H, CONH), 5.67 (t, 1 H, J = 8 Hz), 3.90 (t, 2 H, J = 6 Hz), 3.73 (s, 3 H), 3.20 (d, 1 H, J = 8 Hz, CH₂CO), 1.8-1.55 (m, 2 H) 1.5-1.3 (m, 2 H), 0.91 (t, 3 H, J = 7 Hz); ¹³C NMR (DMSO-d₆) δ 171.2, 167.6, 148.8, 147.7, 134.5, 131.6, 131.2, 123.0, 119.4, 112.8, 111.4, 67.8, 55.5, 50.3, 36.9, 30.7, 18.6, 13.6.

### EXAMPLE 43

A mixture of 3-nitrophthalic anhydride (1.54 g, 8.0 mmol) and 3-amino-3-(4'-methoxyphenyl)propionic acid (1.56 g, 8.0 mmol) in 15 mL of acetic acid was refluxed for 3.5 hours under nitrogen. The reaction was cooled and the solution partially concentrated. The slurry was filtered and the solid was dried to afford 2.34g (79%) of 3-(4'-methoxyphenyl)-3-(3'-nitrophthalimido)propionic acid as a white powder: mp 178-180°C; ¹H NMR (DMSO-d₆, 250 MHz) δ 8.07-8.02 (m, 3 H), 7.38 (d, 2 H, J = 8.7), 6.90 (d, 2H, J = 8.7, 5.68-5.07 (m, 1H), 3.72 (s, 3H), 3.48-3.22 (m, 2H); ¹³C NMR (DMSO-d₆) δ 171.6, 165.7, 163.0, 158.8, 144.4, 136.4, 133.0, 130.2, 128.6, 128.5, 127.0, 122.4, 113.9, 55.1, 50.0, 35.8. Anal. Calcd for C₁₈H₁₄N₂O₇. Theoretical: C, 58.38; H,3.81; N, 7.56. Found: C, 58.18; H, 3.79; N, 7.36.

### EXAMPLE 44

A mixture of 4.5-dichlorophthalic anhydride (0.91, 4.19 mmol) and 3-amino-3-(4'-methoxyphenyl)propionic acid in 10 mL acetic acid was stirred under nitrogen for 6 hours. The reaction was cooled and removed some of the solvent. The slurry was filtered and the solid was dried to afford 1.20g (61%) of 3-(4',5'-dichlorophthalimido)-3-(4'-methoxyphenyl)propionic acid as a white powder. mp 182-185°C; ¹H NMR (DMSO-d₆, 250 MHz) δ 8.19(s, 2H), 7.34 (d, 2 H, J = 8.7), 6.90 (d, 2H, J = 8.7), 5.61(t, 1H, J= 7.8), 3.72 (s, 3H), 3.50-3.20 (m, 2H). ¹³C NMR (DMSO-d₆) δ 171.6, 165.8, 158.8, 137.6, 131.0, 130.4, 128.4, 125.4, 113.9, 55.1, 50.0, 35.8. Anal. Calcd for C₁₈H₁₄N₂O₇. Theoretical: pending

### EXAMPLE 45

A mixture of 3-phthalimido-3-(3',4'-dimethoxyphenyl)propionic acid (0.86 g, 2.41 mmol) and carbonyldiimidazole (0.43 g, 2.65 mmol) with trace amount of 4-dimethylaminopyridine in 10 mL of tetrahydrofuran under nitrogen was stirred for 30 in at room temperature, then 0.23 mL (2.41 mmol) of 3-pyridylcarbinol was added to the above solution. After 1 hour, the reaction mixture was concentrated to an oil. The oil was dissolved in 25 mL of ethylacetate and the mixture was extracted with water (3x25 mL). The organic layer was dried over sodium sulfate and concentrated to afford the crude product as a light yellow oil. The crude product was then purified by flash chromatography (silica gel, MeOH/CH₂Cl₂, 0-2%, (v/v)) to afford 0.54 g (50%) of 3-Pyridinemethyl 3-phthalimido-3-(3',4'-dimethoxyphenyl)propionate as a light yellow foam: ¹H NMR (dmso-d₆, 250 MHz) δ 8.4-8.5 (m, 2 H), 7.84 (s, 4 H, Ar), 7.5-7.6 (m, 1 H), 7.2-7.3 (m, 1 H), 6.7-7.1 (m, 3 H, Ar), 5.65 (dd, 1 H, J₁ = 6 Hz, J₂ = 9.6 Hz), 5.09 (s, 2 H), 3.74 (s, 6 H), 3.4-3.7 (m, 2 H); ¹³C NMR (dmso-d₆) δ 170.1, 167.6, 149.2, 148.6, 148.4, 135.7, 134.7, 131.4, 131.0, 130.8, 123.3, 123.2, 119.3, 111.7, 111.0, 63.4, 55.5, 55.4, 49.9, 35.9. Anal. Calcd for C₂₅H₂₂N₂O₆. Theoretical C, 67.26; H, 4.97; N, 6.27. Found C, 67.06; H, 4.99; N, 6.20.

### EXAMPLE 46

A mixture of 3-phthalimido-3-(3',4'-dimethoxyphenyl) propionic acid (0.60 g, 1.69 mmol), carbonyldiimidazole (0.28 g, 1.77 mmol) and trace amount of 4-dimethylaminopyridine in 10 mL of tetrahydrofuran was stirred at room temperature under nitrogen for 30 minutes. To the reaction mixture was added 3-aminomethylpyridine (0.18 mL, 1.77 mmol). The reaction mixture was stirred for 20 minutes, then 10 mL of water was added and the tetrahydrofuran was removed under reduced pressure. The resulting slurry was filtered, the solid was washed with water, and dried *in vacuo* (60 °C, < 133Nm⁻² (<1 mm)) to afford 0.57 g (76%) of N-3-methylpyridyl 3-phthalimido-3-(3',4'-dimethoxyphenyl)propionamide as a white powder: mp 171.2-172.4 °C; ¹H NMR (dmso-d₆, 250 MHz) δ 8.69 (t, 1 H, J = 6 Hz), 8.36 (m, 2H), 7.85 (s, 4 H, Ar), 6.8-7.4 (m, 5 H), 5.71 (t, 1 H, J = 8 Hz), 4.22 (d, 2 H, J = 5.2 Hz), 3.73 (s, 3 H), 3.71 (s, 3 H), 3.31 (d, 2 H, J= 8 Hz); ¹³C NMR (dmso-d₆) δ 169.4, 167.7, 148.5, 148.3, 147.9, 134.7, 134.6, 134.5, 131.4, 131.2, 123.1, 119.5, 111.6, 111.2, 55.5, 55.4, 50.6, 39.6, 37.4. Anal. Calcd for C₂₅H₂₃N₃O₅. Theoretical C, 67.41; H, 5.20; N, 9.43. Found C, 67.35; H, 5.14; N, 9.34.

### EXAMPLE 47

To a stirred solution of 3-phthalimido-3-(3',4'-dichlorophenyl)propionic acid (1.10 g, 3.02 mmol) in 20 mL of tetrahydrofuran at room temperature under nitrogen was added carbonyldiimidazole (0.51 g, 3.17 mmol) and a catalytic amount of 4-dimethylaminopyridine. The mixture was stirred for 45 minutes and then concentrated ammonium hydroxide (0.21 mL, 3.2 mmol) was added. The reaction mixture was stirred for 10 minutes and then the tetrahydrofuran was removed under reduced pressure. To the resulting mixture was added 20 mL of water, a light yellow oil was formed. To the mixture was added 3 mL of ether, the mixture was stirred at room temperature for 1 hour. The resulting slurry was filtered, the solid was washed with water and air-dried to afford 0.73 g of the crude product as a white solid. The crude product was purified by flash chromatography (silica gel, hexane/CH₂Cl₂, 22-0% (v/v)) to afford 0.39 g (36%) of 3-phthalimido-3-(3',4'-dichlorophenyl)propionamide as a white powder: ¹H NMR (dmso-d₆, 250 MHz) δ 7.83 (m, 4 H, Ar), 7.35-7.75 (m, 4 H), 6.93 (br s, 1 H), 5.72 (t, 1 H, J = 8 Hz), 3.25 (dd, 1 H, J₁ = 8 Hz, J₂ = 15 Hz), 3.14 (dd, 1 H, J₁ = 8 Hz, J₂ = 15 Hz); ¹³C NMR (dmso-d₆) δ 170.8, 167.6, 140.2, 134.6, 131.2, 131.0, 130.7, 130.3, 129.2, 127.7, 123.2, 49.3, 36.5. Anal. Calcd for C₁₇H₁₂N₂O₃Cl₂. Theoretical C, 54.69; H, 3.54; N, 7.53. Found C, 54.69; H, 3.38; N, 7.15.

### EXAMPLE 48

To 150 mL of stirred methanol at 0 °C under nitrogen was slowly added thionyl chloride (14.2 mL, 194.4 mmol). To the reaction mixture was then added 3-amino-3-(3',4'-dimethoxyphenyl)propionic acid (15.5 g, 64.8 mmol). The reaction mixture was stirred at 0 °C for 30 minutes and then allowed to warm to room temperature, and stirred overnight. The reaction solution was concentrated to an oil and then diluted with 200 mL of CH₃OH/Et₂O (1/3) and stirred. The resulting slurry was filtered and the solid was washed with a copious amount of ether. The solid was dried *in vacuo* (60 °C, < 133Nm⁻² (< 1 mm)) to afford 18.3 g (66%) of methyl 3-amino-3-(3',4'-dimethoxyphenyl)propionate hydrochloride as a white powder: ¹ H NMR (dmso-d₆, 250 MHz) δ 8.59 (br s, 3 H, NH₃), 6.9-7.3 (m, 3 H, Ar), 4.52 (overlapping dd, 1 H), 3.77 (s, 3 H, OCH₃), 3.75 (s, 3 H, OCH₃), 3.57 (s, 3 H, OCH₃), 3.16 (dd, 1 H, J₁ = 6 Hz, J₂ = 16 Hz), 2.98 (dd, 1 H, J₁ = 8 Hz, J₂ = 16 Hz); ¹³C NMR (dmso-d₆) δ 169.6, 149.0, 129.0, 120.0, 111.5, 111.4, 55.7, 55.5, 51.7, 50.8, 38.5. Anal. Calcd for C₁₂H₁₈NO₄Cl. Theoretical C, 52.27; H, 6.58; N, 5.08. Found C, 52.44; H, 6.53; N, 5.01.

### EXAMPLE 49

A mixture of methy 3-amino-3-(3',4'-dimethoxyphenyl)propionate hydrochloride (1.38 g, 5.00 mmol), .sodium carbonate (0.53 g, 5.00 mmol), and N-carboethoxyphthalimide (1.10 g, 5.0 mmol) in 40 mL of CH₃CN/H₂O (1/1) was stirred for 1 hour at room temperature. The reaction solution was then partially concentrated under reduced pressure to remove the acetonitrile. This afforded a white gum in water. To the mixture was then added 5 mL of ether and the mixture was stirred for 2 hours. The resulting slurry was filtered, the solid was washed with a copious amount of water and air-dried overnight to afford 1.69 g (92%) of methyl 3-phthalimido-3-(3',4'-dimethoxyphenyl)propionate as a white solid: mp 114-116 °C; ¹H NMR (dmso-d₆, 250 MHz) δ 7.80-7.95 (m, 4 H, Ar), 6.80-7.10 (m, 3 H, Ar), 5.65 (dd, 1 H, J₁ = 7 Hz, J₂ = 9 Hz), 3.74 (s, 3 H, OCH₃), 3.72 (s, 3 H, OCH₃), 3.55 (s, 3 H, OCH₃), 3.30-3.67 (m, 2 H); ¹³C NMR (dmso-d₆) δ 170.8, 167.6, 148.6, 148.4, 134.7, 131.1, 131.0, 123.2, 119.3, 111.7, 111.0, 55.5, 51.6, 49.9, 35.6. Anal. Calcd for C₂₀H₁₉NO₆. Theoretical C, 65.03; H, 5.18; N, 3.79. Found C, 65.17; H, 5.14; N, 3.75. HPLC 99%.

### REFERENTIAL EXAMPLE 50

To a stirred solution of benzaldehyde (1.58 mL, 15.5 mmol) in 10 mL of absolute ethanol at room temperature under nitrogen was added (R)-_{α}-methylbenzylamine (2.0 mL, 15.51 mmol, 99% ee.). The reaction mixture was stirred for 3 hours. The reaction solution was then dried over magnesium sulfate and diluted to a 60 mL volume with EtOH. Ethanol washed Raney-Ni (1.5 g) was added and the resulting suspension was treated with 58 psi of H₂ in a Parr Type Shaker. After 1 day, additional Raney-Ni (-1 g) and 30 mL of ethanol were added and the hydrogenolysis continued for 3 days. The reaction mixture was filtered through Celite to remove the catalyst and concentrated to afford 3.11 g (95%) of N-benzyl (R)-α-methylbenzylamine as a pale yellow oil contaminated with 5% of benzyl alcohol and (R)-α-methylbenzylamine; ¹H NMR (dmso-d₆, 250 MHz) δ 7.1-7.5 (m, 10 H, Ar), 3.68 (q, 1 H, J = 6.6 Hz), 3.48 (dd, 2 H, J₁ = 13.6 Hz, J₂ = 20.5 Hz), 1.26 (d, 3 H, J = 6.6 Hz, CH₃); ¹³C NMR (dmso-d₆) δ 146.1, 141.0, 128.2, 128.0, 127.8, 126.5, 126.4, 56.7, 50.6, 24.5. This mixture was used directly in the next reaction.

### REFERENTIAL EXAMPLE 51

To stirred solution of N-benzyl (R)-α-methylbenzylamine (1.9 g, 9.0 mmol) in 50 mL of tetrahydrofuran at 0 °C under nitrogen was added n-butyl lithium (1.6 M in hexanes; 9.0 mmol). The resulting red solution was stirred at 0 °C for 15 minutes and then cooled to -78 °C. To the reaction mixture was then dropwise added methyl trans-3-(3',4'-dimethoxyphenyl)propion-2-enate (1.33 g, 6.0 mmol) in 20 mL tetrahydrofuran and the mixture was stirred for 15 minutes at -78 °C to afford a yellow solution. The reaction was quenched by the addition of saturated ammonium chloride (20 mL). The mixture was allowed to warm to room temperature and poured into 40 mL of saturated sodium chloride (aq). The mixture was extracted with ether (2x 60 mL) and the combined organic layers dried (MgSO₄) and concentrated to afford 3.35 g of crude product as a yellow oil. The oil was purified by flash chromatography (silica gel, hexane/CH₂Cl₂, 30-0%, (v/v)) to afford 1.24 g (48%) of methyl (S)-N-Benzyl-N-(R)-α-methylbenzyl-3-(3',4'-dimethoxyphenyl)propionate adduct as colorless oil: ¹H NMR (dmso-d₆, 250 MHz) δ 6.7-7.5 (m, 13 H, Ar), 3.9-4.2 (m, 2 H), 3.78 (s, 3 H, OCH₃), 3.73 (s, 3 H, OCH₃), 3.65 (s, 2 H), 3.43 (s, 3 H, OCH₃), 2.6-2.9 (m, 2 H), 1.03 (d, 3 H, J = 7 Hz); ¹³C NMR (dmso-d₆) δ 171.6, 148.3, 147.8, 144.6, 141.5, 133.6, 128.1, 128.0, 127.7, 127.5,126.7, 126.4, 119.6, 111.9, 111.2, 58.2, 56.4, 55.4, 55.3, 51.1, 49.7, 35.6, 17.1.

### REFERENTIAL EXAMPLE 52

Debenzylation of the above pure adduct was done following the literature procedure of S. G. Davies and O. Ichihara *(Tetrahedron Asymmetry* 1991, **2**, 183.). To a stirred solution methyl (S)-3-(N-benzyl-N-(R)-a-methylbenzylamino)-3-(3',4'-dimethoxyphenyl)propionate (1.20 g, 2.77 mmol) in a mixture of MeOH (20 mL), water (2 mL) and acetic acid (0.5 mL) was added 20% palladium hydroxide on charcoal. The reaction mixture was treated with hydrogen (54 psi) at room temperature for 23 h on a Parr Type Shaker. The reaction mixture was filtered through Celite and then concentrated to afford the product as an acetate salt. The salt was dissolved in 10 mL of water, stirred with 0.7 mL 4 N HCl and then concentrated to a white solid. The solid was diluted with 40 mL of ether and stirred for 20 min. The slurry was filtered and the solid was dried *in vacuo* (room temperature, < 133Nm⁻² (< 1 mm)) to afford 0.57 g (75%) of methyl (S)-3-amino-3-(3',4'-dimethoxyphenyl)propionate hydrochloride as a white solid: HPLC 96% ee (Chiral Crownpack CR+ column); ¹H NMR (dmso-d₆, 250 MHz) δ 8.73 (br s, 3 H, NH₃), 6.90-7.40 (m, 3 H, Ar), 4.51 (dd, 1 H, J₁ = 6 Hz, J₂ = 8 Hz), 3.77 (s, 3 H, OCH₃), 3.75 (s, 3 H, OCH₃), 3.56 (s, 3 H, OCH₃), 3.2 (dd, 1 H, J₁ = 6 Hz, J₂ = 16 Hz), 3.0 (dd, 1 H, J₁ = 8 Hz, J₂ = 16 Hz); ¹³C NMR (dmso-d₆) δ 169.6, 149.0, 148.7, 129.0, 120.0, 111.5, 111.4, 55.7, 55.5, 51.7, 50.8, 38.6. Anal.Calcd for C₁₂H₁₈NO₄Cl-0.48 H₂O. Theoretical C, 50.67; H, 6.72; N, 4.92. Found C, 50.67; H, 6.46; N, 4.83.

### EXAMPLE 53

Prepared as described earlier for methyl 3-phthalimido-3-(3',4'-dimethoxyphenyl)propionate from methyl (S)-3-amino-3-(3',4'-dimethoxyphenyl)propionate (0.45 g, 1.63 mmol), sodium carbonate (0.17 g, 1.63 mmol) and N-carboethoxyphthalimide (0.36 g, 1.63 mmol). Methyl (S)-3-phthalimido-3-(3',4'-dimethoxyphenyl)propionate was obtained as a white powder, 0.51 g (85%): ¹H NMR (dmso-d₆, 250 MHz) δ 7.87 (br s, 4 H, Ar), 6.80-7.10 (m, 3 H, Ar), 5.65 (dd, 1 H, J₁ = 7 Hz, J₂ = 9 Hz), 3.73 (s, 3 H, OCH₃), 3.72 (s, 3 H, OCH₃), 3.55 (s, 3 H, OCH₃), 3.30-3.67 (m, 2 H); ¹³C NMR (dmso-d₆) δ 170.8, 167.7, 148.6, 148.4, 134.7, 131.1, 131.0, 123.2, 119.3, 111.7, 111.0, 55.5, 51.6, 49.9, 35.6. Anal.Calcd for C₂₀H₁₉NO₆. Theoretical C, 65.03; H, 5.18; N, 3.79. Found C, 64.94; H, 5.29; N, 3.86. HPLC 97%.

### REPERENTIAL EXAMPLE 54

Prepared as described earlier for N-benzyl-(R)-α-methylbenzylamine from benzaldehyde (3.94 mL, 38.8 mmol) and (S)-a-methylbenzylamine (5.0 mL, 38.8 mmol, 96% ee.) to afford 7.88 g (96%) of N-benzyl-(S)-α-methylbenzylamine as an oil contaminated with ⁻ 5% of benzyl alcohol and (S)-a-methylbenzylamine: ¹H NMR (dmso-d₆, 250 MHz) δ 7.15-7.45 (m, 10 H, Ar), 3.69 (q, 1 H, J = 6.5 Hz), 3.48 (dd, 2 H, J₁ = 13.6 Hz, J₂ = 20.9 Hz), 2.45 (br s, 1 H, NH), 1.26 (d, 3 H, J = 6.5 Hz, CH₃) ; ¹³C NMR (dmso-d₆) δ 146.0, 141.0, 128.1, 128.0, 127.8, 126.4, 126.3, 56.7, 50.6, 24.5. This mixture was directly used in the next reaction.

### REFERENTIAL EXAMPLE 55

Prepared as described earlier for methyl (S)-3-(N-benzyl-N-(R)-α-methylbenzyl)-3-(3',4'-dimethoxyphenyl)propionate from butyl lithium (1.6 M in hexanes; 8.44 mmol), N-benzyl-(S)-a-methylbenylamine (1.78 g, 8.44 mmol) and 3-(3',4'-dimethoxyphenyl)propyl-2-enate (1.50 g, 6.75 mmol) to afford 3.7 g of the crude product as a yellow oil. The oil was purified by flash chromatography (silica gel, ether/hexane, 20/80) to afford 0.57 g (20%) of methyl (R)-3-(N-benzyl-N-(S)-a-methylbenzylamino)-3-(3',4'-dimethoxyphenyl)propionate as a colorless oil; ¹H NMR (dmso-d₆, 250 MHz) δ 6.80-7.50 (m, 13 H, Ar), 4.15 (dd, 1 H, J₁ =6 Hz, J₂ = 9 Hz), 4.04 (q, 1 H, J = 7 Hz), 3.78 (s, 3 H, OCH₃), 3.73 (s, 3 H, OCH₃), 3.69 (s, 2 H), 3.43 (s, 3 H, OCH₃), 2.87 (dd, 1 H, J₁ =6 Hz, J₂ = 15 Hz), 2.67 (dd, 1 H, J₁ = 9 Hz, J₂ = 15 Hz), 1.04 (d, 3 H, J = 7 Hz, CH₃); ¹³C NMR (dmso-d₆) δ 171.6, 148.4, 147.8, 144.7, 141.5, 133.6, 128.1, 128.0, 127.8, 127.5,126.7, 126.4, 119.6, 111.9, 111.2, 58.2, 56.4, 55.4, 55.3, 51.1, 49.7, 35.6, 17.1.

### REFERENTIAL EXAMPLE 56

Debenzylation of the above adduct was done following the literature procedure of S. G. Davies and O. Ichihara (*Tetrahedron Asymmetry* 1991, **2**, 183.). A solution of methyl (R)-3-(N-benzyl-N-(S)-a-methylbenzylamino)-3-(3',4'-dimethoxyphenyl)propionate (0.57 g, 1.3 mmol) in MeOH (10 mL), water (1 mL) and acetic acid (0.25 mL) in the presence of 20% palladium hydroxide on charcoal was treated with hydrogen (59 psi) at room temperature for 23 h in a Parr Type Shaker. The mixture was filtered through Celite and then concentrated to afford the primary amine in an acetate salt, which was dissolved in 10 mL of water, stirred with 0.32 mL (4 N) HCl and concentrated to a white solid. To the solid was added 10 mL of ether and the mixture stirred for 30 min. The slurry was filtered, the solid was dried *in vacuo* (room temperate, < 133Nm⁻² (< 1 mm)) to afford 0.32 g (90%) of methyl (R)-3-amino-3-(3',4'-dimethoxyphenyl)propionate hydrochloride as a white powder: Chiral HPLC (Crownpak Cr+ Column 92% ee; ¹H NMR (dmso-d₆, 250 MHz) δ 8.61 (br s, 3 H, NH₃), 6.90-7.30 (m, 3 H, Ar), 4.53 (br s, 1 H), 3.77 (s, 3 H, OCH₃), 3.75 (s, 3 H, OCH₃), 3.57 (s, 3 H, OCH₃), 3.16 (dd, 1 H, J₁ = 6 Hz, J₂ = 16 Hz), 2.98 (dd, 1 H, J₁ = 8 Hz, J₂ = 16 Hz); ¹³C NMR (dmso-d₆) δ 169.5, 149.0, 148.6, 128.9, 119.8, 111.4, 111.2, 55.6, 55.4, 51.7, 50.7, 38.4. Anal.Calcd for C₁₂H₁₈NO₄Cl @0.48 H₂O. Theoretical C, 50.67; H, 6.72; N, 4.92. Found C, 50.66; H, 6.54; N, 4.81.

### EXAMPLE 57

Prepared as described earlier for methyl 3-phthalimido-3-(3',4'-dimethoxyphenyl)propionate from methyl (3R)-3-amino-3-(3',4'-dimethoxyphenyl)propionate (0.25 g, 0.91 mmol), sodium carbonate (0.10 g, 0.91 mmol) and N-carboethoxyphthalimide (0.20 g, 0.91 mmol). Methyl (3R)-3-phthalimido-3-(3',4'-dimethoxyphenyl)propionate was obtained as a white powder, 0.29 g (88%); ¹H NMR (dmso-d₆, 250 MHz) δ 7.87 (br s, 4 H, Ar), 6.80-7.10 (m, 3 H, Ar), 5.64 (dd, 1 H, J₁ = 7 Hz, J₂ = 9 Hz), 3.73 (s, 3 H, OCH₃), 3.72 (s, 3 H, OCH₃), 3.55 (s, 3 H, OCH₃), 3.30-3.67 (m, 2 H); ¹³C NMR (dmso-d₆) δ 170.8, 167.6, 148.6, 148.4, 134.7, 131.1, 131.0, 123.2, 119.2, 111.7, 111.0, 55.5, 51.6, 49.9, 35.6. Anal.Calcd for C₂₀H₁₉NO₆ @0.80 H₂O. Theoretical C, 62.60; H, 4.99; N, 3.69. Found C, 62.60; H, 4.93; N, 3.69. HPLC 99.9%.

### EXAMPLE 58

Tablets, each containing 50 mg of active ingredient, can be prepared in the following manner:

| Constituents (for 1000 tablets) | |
|---|---|
| active ingredient | 50.0 g |
| lactose | 50.7 g |
| wheat starch | 7.5 g |
| polyethylene glycol 6000 | 5.0 g |
| talc | 5.0 g |
| magnesium stearate | 1.8 g |
| demineralized water | q.s. |

The solid ingredients are first forced through a sieve of 0.6 mm mesh width. The active ingredient, the lactose, the talc, the magnesium stearate and half of the starch then are mixed. The other half of the starch is suspended in 40 mL of water and this suspension is added to a boiling solution of the polyethylene glycol in 100 mL of water. The resulting paste is added to the pulverulent substances and the mixture is granulated, if necessary with the addition of water. The granulate is dried overnight at 35°C, forced through a sieve of 1.2 mm mesh width and compressed to form tablets of approximately 6 mm diameter which are concave on both sides.

### EXAMPLE 59

Tablets, each containing 100 mg of active ingredient, can be prepared in the following manner:

| Constituents (for 1000 tablets) | |
|---|---|
| active ingredient | 100.0 g |
| lactose | 100.0 g |
| wheat starch | 47.0 g |
| magnesium stearate | 3.0 g |

All the solid ingredients are first forced through a sieve of 0.6 mm mesh width. The active ingredient, the lactose, the magnesium stearate and half of the starch then are mixed. The other half of the starch is suspended in 40 mL of water and this suspension is added to 100 mL of boiling water. The resulting paste is added to the pulverulent.substances and the mixture is granulated, if necessary with the addition of water. The granulate is dried overnight at 35°C, forced through a sieve of 1.2 mm mesh width and compressed to form tablets of approximately 6 mm diameter which are concave on both sides.

### EXAMPLE 60

Tablets for chewing, each containing 75 mg of active ingredient, can be prepared in the following manner:

| Composition (for 1000 tablets) | |
|---|---|
| active ingredient | 75.0 g |
| mannitol | 230.0 g |
| lactose | 150.0 g |
| talc | 21.0 g |
| glycine | 12.5 g |
| stearic acid | 10.0 g |
| saccharin | 1.5 g |
| 5% gelatin solution | q.s. |

All the solid ingredients are first forced through a sieve of 0.25 mm mesh width. The mannitol and the lactose are mixed, granulated with the addition of gelatin solution, forced through a sieve of 2 mm mesh width, dried at 50°C and again forced through a sieve of 1.7 mm mesh width. The active ingredient, the glycine and the saccharin are carefully mixed, the mannitol, the lactose granulate, the stearic acid and the talc are added and the whole is mixed thoroughly and compressed to form tablets of approximately 10 mm diameter which are concave on both sides and have a breaking groove on the upper side.

### EXAMPLE 61

Tablets, each containing 10 mg of active ingredient, can be prepared in the following manner:

| Composition (for 1000 tablets) | |
|---|---|
| active ingredient | 10.0 g |
| lactose | 328.5 g |
| corn starch | 17.5 g |
| polyethylene glycol 6000 | 5.0 g |
| talc | 25.0 g |
| magnesium stearate | 4.0 g |
| demineralized water | q.s. |

The solid ingredients are first forced through a sieve of 0.6 mm mesh width. Then the active ingredient, lactose, talc, magnesium stearate and half of the starch are intimately mixed. The other half of the starch is suspended in 65 mL of water and this suspension is added to a boiling solution of the polyethylene glycol in 260 mL of water. The resulting paste is added to the pulverulent substances, and the whole is mixed and granulated, if necessary with the addition of water. The granulate is dried overnight at 35°C, forced through a sieve of 1.2 mm mesh width and compressed to form tablets of approximately 10 mm diameter which are concave on both sides and have a breaking notch on the upper side.

### EXAMPLE 62

Gelatin dry-filled capsules, each containing 100 mg of active ingredient, can be prepared in the following manner:

| Composition (for 1000 capsules) | |
|---|---|
| active ingredient | 100.0 g |
| microcrystalline cellulose | 30.0 g |
| sodium lauryl sulphate | 2.0 g |
| magnesium stearate | 8.0 g |

The sodium lauryl sulphate is sieved into the active ingredient through a sieve of 0.2 mm mesh width and the two components are intimately mixed for 10 minutes. The microcrystalline cellulose is then added through a sieve of 0.9 mm mesh width and the whole is again intimately mixed for 10 minutes. Finally, the magnesium stearate is added through a sieve of 0.8_mm width and, after mixing for a further 3 minutes, the mixture is introduced in portions of 140 mg each into size 0 (elongated) gelatin dry-fill capsules.

A 0.2% injection or infusion solution can be prepared, for example, in the following manner:

| | |
|---|---|
| active ingredient | 5.0 g |
| sodium chloride | 22.5 g |
| phosphate buffer pH 7.4 | 300.0 g |
| demineralized water | to 2500.0 mL |

The active ingredient is dissolved in 1000 mL of water and filtered through a microfilter. The buffer solution is added and the whole is made up to 2500 mL with water. To prepare dosage unit forms, portions of 1.0 or 2.5 mL each are introduced into glass ampoules (each containing respectively 2.0 or 5.0 mg of active ingredient).

## Claims

1. A compound of the formula: 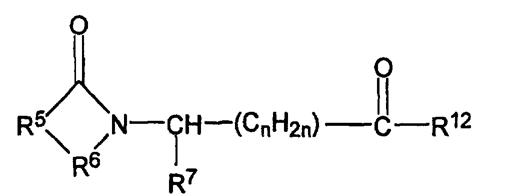 in which
R⁵ is *(i) o*-phenylene, unsubstituted or substituted with 1 to 3 substituents each selected independently from nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, or *(ii)* the divalent residue of pyridine, pyrrolidine, imidizole, naphthalene, or thiophene, wherein the divalent bonds are on vicinal ring carbon atoms;
R⁶ is -CO-, -CH₂- or -SO₂-;
R⁷ is *(i)* pyridyl, *(ii)* phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, (*iii*) benzyl substituted with one to three substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, *(iv)* naphthyl, *(v)* benzyloxy, or (*vi*) imidazol-4-ylmethyl;
R¹² is -OH, alkoxy of 1 to 12 carbon atoms, -O-CH₂-pyridyl, -O-benzyl, or 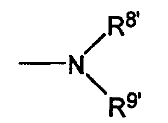 where *n* has a value of 0, 1, 2, or 3;
R⁸ is hydrogen or alkyl of 1 to 10 carbon atoms; and
R⁹ is hydrogen, alkyl of 1 to 10 carbon atoms, -CH₂-pyridyl, benzyl, -COR¹⁰, or -SO₂R¹⁰ in which R¹⁰ is hydrogen, alkyl of 1 to 4 carbon atoms, or phenyl.

2. The compound according to claim 1 wherein R¹² is -O-methyl.

3. The compound of claim 2 wherein R⁵ is *o*-phenylene unsubstituted or substituted with 1 to 3 substituents each selected independently from nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo.

4. The compound according to claim 3 wherein:
R⁵ is *o*-phenylene;
R⁶ is -CO-;
R⁷ is *(i)* pyridyl, *(ii)* phenyl substituted with cyano, trifluoromethyl, carbethoxy, carbomethoxy, carbopropoxy, acetyl, carbamoyl, acetoxy, carboxy, hydroxy, alkyl of 2 to 10 carbon atoms, alkoxy of 1 to 10 carbon atoms, or halo, *(iii)* phenyl substituted with 2 or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, *(iv)* benzyl substituted with 1 to 3 substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, *(v)* naphthyl, *(vi)* benzyloxy, or *(vii)* imidazol-4-ylmethyl; and
where n has a value of 1.

5. The compound according to claim 4 which is methyl [3-phthalimido-3-(3,4-dimethoxyphenyl)]propionate.

6. The compound according to claim 4 which is methyl [3-phthalimido-3-(3,4-diethoxyphenyl)]propionate.

7. The compound of claim 2 wherein R⁶ is -CH₂-.

8. The compound of claim 7 wherein R⁵ is *o*-phenylene unsubstituted or substituted with 1 to 3 substituents each selected independently from nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo.

9. The compound according to claim 1 wherein R⁶ is -CH₂-.

10. The compound of claim 9 wherein R⁵ is *o*-phenylene unsubstituted or substituted with 1 to 3 substituents each selected independently from nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo.

11. The compound according to claim 1 wherein:
R⁵ is *o*-phenylene;
R⁶ is -CO-;
R⁷ is *(i)* pyridyl, *(ii)* phenyl substituted with cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 2 to 10 carbon atoms, or halo, *(iii)* phenyl substituted with 2 or more substituents each selected independent of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, *(iv)* benzyl substituted with 1 to 3 substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, *(v)* naphthyl, *(vi)* benzyloxy, or *(vii)* imidazol-4-ylmethyl;
R¹² is -OH; and
where n has a value of 1.

12. The compound according to claim 1 wherein:
R⁵ is *o*-phenylene;
R⁶ is -CO-;
R⁷ is *(i)* pyridyl, *(ii)* phenyl substituted with cyano, trifluoromethyl, carbethoxy, carbomethoxy, carbopropoxy, acetyl, carbamoyl, acetoxy, carboxy, hydroxy, alkyl of 1 to 10 carbon atoms, alkoxy of 2 to 10 carbon atoms, or halo, *(iii)* phenyl substituted with 2 or more substituents each selected independent of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, *(iv)* benzyl substituted with 1 to 3 substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, *(v)* naphthyl, *(vi)* benzyloxy, or *(vii)* imidazol-4-ylmethyl;
R¹² is ethoxy; and
where n has a value of 1.

13. The compound according to claim 1 wherein: 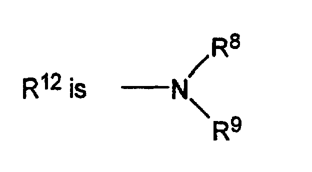 where R⁸ is hydrogen or alkyl of 1 to 10 carbon atoms;
R⁹ is hydrogen, alkyl of 1 to 10 carbon atoms, -CH₂-pyridyl, benzyl, -COR¹⁰, or -SO₂R¹⁰ in which R¹⁰ is hydrogen, alkyl of 1 to 10 carbon atoms, or phenyl.

14. The compound according to claim 13 wherein:
R⁵ is *o*-phenylene;
R⁶ is -CO-;
R⁷ is *(i)* pyridyl, *(ii)* phenyl substituted with one or more substituents each selected independent of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, *(iii)* benzyl substituted with 1 to 3 substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, *(iv)* naphthyl, *(v)* benzyloxy, or *(vi)* imidazol-4-ylmethyl; and
where n has a value of 1.

15. The compound according to claim 14 which is 3-phthalimido-3-(4-methoxyphenyl)propanamide.

16. The compound according to claim 14 which is 3-phthalimido-3-(3,4-dimethoxyphenyl)propanamide.

17. The compound according to claim 14 which is 3-phthalimido-3-(3,4-diethoxyphenyl)propanamide.

18. The compound according to claim 1 wherein, R⁵ is amino substituted *o*-phenylene.

19. The compound according to claim 18 which is 3-(amino-phthalimido)-3-(3,4-dimethoxyphenyl)propanamide.

20. The compound according to claim 18 which is 3-(amino-phthalimido)-3-(3,4-diethoxyphenyl)propanamide.

21. The compound according to claim 18 which is methyl [3-(amino-phthalimido)-3-(3,4-dimethoxyphenyl)]propionate.

22. The compound according to claim 18 which is methyl [3-(amino-phthalimido)-3-(3,4-diethoxyphenyl)]propionate.

23. A compound according to any of claims 1 to 22 for use in therapy.

24. A pharmaceutical composition comprising an amount of a compound according to any one of claims 1 to 22 effective upon single or multiple dosage to inhibit TNFα.

25. Use of a compound in the manufacture of a medicament for the treatment of a disease which is treatable by inhibiting formation of or reducing levels of TNFα in a mammal wherein the compound is of the formula: 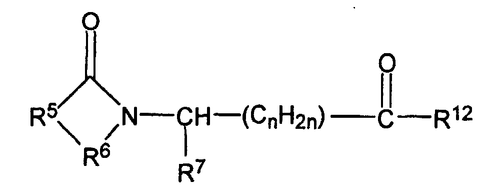 in which R⁵ is (*i*) *o*-phenylene, unsubstituted or substituted with 1 to 3 substituents each selected independently from nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, or (*ii*) the divalent residue of pyridine, pyrrolidine, imidizole, naphthalene, or thiophene, wherein the divalent bonds are on vicinal ring carbon atoms;
R⁶ is -CO-, -CH₂- or -SO₂- ;
R⁷ is (*i*) straight, branched, or cyclic alkyl of 1 to 12 carbon atoms, (*ii*) pyridyl, (*iii*) phenyl or phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, (i*v*) benzyl unsubstituted or substituted with one to three substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, *(v)* naphthyl, *(vi)* benzyloxy, or (*vii*) imidazol-4-ylmethyl;
R¹² is -OH, alkoxy of 1 to 12 carbon atoms, -O-CH₂-pyridyl, -O-benzyl, or 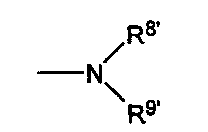 *n* has a value of 0, 1, 2, or 3;
R⁸ is hydrogen or alkyl of 1 to 10 carbon atoms; and
R⁹ is hydrogen, alkyl of 1 to 10 carbon atoms, -CH₂-pyridyl, benzyl, -COR¹⁰, or -SO₂R¹⁰ in which R¹⁰ is hydrogen, alkyl of 1 to 4 carbon atoms, or phenyl.

26. Use of a compound according to claim 25 wherein R¹² is

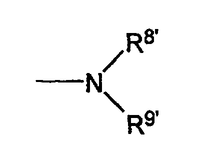

27. Use of a compound according to claim 26 wherein in the compound:
R⁵ is *o*-phenylene;
R⁶ is -CO-; and
where n has a value of 1.

28. Use of a compound according to claim 25 wherein in the compound:
R⁵ is *o*-phenylene;
R⁶ is -CO-;
R¹² is methoxy; and
where n has a value of 1.

29. Use of a compound according to claim 25 wherein in the compound:
R⁵ is *o*-phenylene;
R⁶ is -CO-;
R¹² is -OH; and
where n has a value of 1.

30. Use of a compound according to claim 25 wherein in the compound:
R⁵ is *o*-phenylene;
R⁶ is -CO-;
R¹² is ethoxy; and
where n has a value of 1.

31. Use of a compound according to any one of claims 1 to 22 in the manufacture of a medicament for reducing levels of TNFα in a mammal.

32. Use of a compound according to any one of claims 1 to 22 in the manufacture of a medicament for inhibiting TNFα-activated retrovirus replication in mammals.

## Patentansprüche

1. Verbindung der Formel: 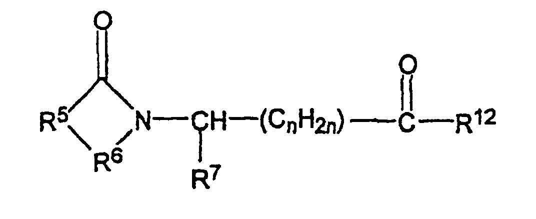 worin
R⁵ *(i) o*-Phenylen, unsubstituiert oder substituiert mit 1 bis 3 Substituenten, jeweils unabhängig voneinander ausgewählt aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 4 Kohlenstoffatomen, Alkoxy mit 1 bis 4 Kohlenstoffatomen oder Halogen oder *(ii)* der zweiwertige Rest von Pyridin, Pyrrolidin, Imidizol, Naphthalin oder Thiophen ist, wobei die zweiwertigen Bindungen an vicinalen Ringkohlenstoffatomen sind;
R⁶ -CO-, -CH₂- oder -SO₂- ist;
R⁷ *(i)* Pyridyl, (*ii*) Phenyl, substituiert mit einem oder mehreren Substituenten, jeweils unabhängig voneinander ausgewählt aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 10 Kohlenstoffatomen, Alkoxy mit 1 bis 10 Kohlenstoffatomen oder Halogen, *(iii)* Benzyl, substituiert mit einem bis drei Substituenten, ausgewählt aus der Gruppe, bestehend aus Nitro, Cyano, Trifluormethyl; Carbethoxy, Carbomethoxy, Carbopropoxy, Acetyl, Carbamoyl, Acetoxy, Carboxy, Hydroxy, Amino, Alkyl mit 1 bis 4 Kohlenstoffatomen, Alkoxy mit 1 bis 4 Kohlenstoffatomen oder Halogen, *(iv)* Naphthyl, *(v)* Benzyloxy oder *(vi)* Imidazol-4-ylmethyl ist;
R¹² -OH, Alkoxy mit 1 bis 12 Kohlenstoffatomen, -O-CH₂-Pyridyl, -O-Benzyl oder 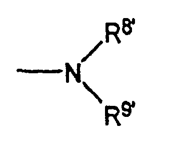 ist,
wobei n einen Wert von 0, 1, 2 oder 3 hat;
R⁸ Wasserstoff oder Alkyl mit 1 bis 10 Kohlenstoffatomen ist; und
R⁹ Wasserstoff, Alkyl mit 1 bis 10 Kohlenstoffatomen, -CH₂-Pyridyl, Benzyl, -COR¹⁰ oder -SO₂R¹⁰ ist, worin R¹⁰ Wasserstoff, Alkyl mit 1 bis 4 Kohlenstoffatomen oder Phenyl ist.

2. Verbindung gemäß Anspruch 1, wobei R¹² -O-Methyl ist.

3. Verbindung nach Anspruch 2, wobei R⁵ *o*-Phenylen ist, unsubstituiert oder substituiert mit 1 bis 3 Substituenten, jeweils unabhängig voneinander ausgewählt aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 4 Kohlenstoffatomen, Alkoxy mit 1 bis 4 Kohlenstoffatomen oder Halogen.

4. Verbindung gemäß Anspruch 3, wobei:
R⁵ *o*-Phenylen ist;
R⁶ -CO- ist;
R⁷ *(i)* Pyridyl, *(ii)* Phenyl, substituiert mit Cyano, Trifluormethyl, Carbethoxy, Carbomethoxy, Carbopropoxy, Acetyl, Carbamoyl, Acetoxy, Carboxy, Hydroxy, Alkyl mit 2 bis 10 Kohlenstoffatomen, Alkoxy mit 1 bis 10 Kohlenstoffatomen oder Halogen, *(iii)* Phenyl, substituiert mit 2 oder mehr Substituenten, jeweils unabhängig voneinander ausgewählt aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 10 Kohlenstoffatomen, Alkoxy mit 1 bis 10 Kohlenstoffatomen oder Halogen, *(iv)* Benzyl, substituiert mit 1 bis 3 Substituenten, ausgewählt aus der Gruppe, bestehend aus Nitro, Cyano, Trifluormethyl, Carbethoxy, Carbomethoxy, Carbopropoxy, Acetyl, Carbamoyl, Acetoxy, Garboxy, Hydroxy, Amino, Alkyl mit 1 bis 4 Kohlenstoffatomen, Alkoxy mit 1 bis 4 Kohlenstoffatomen oder Halogen, *(v)* Naphthyl, *(vi)* Benzyloxy oder *(vii)* Imidazol-4-ylmethyl ist; und
wobei n einen Wert von 1 hat.

5. Verbindung gemäß Anspruch 4, welche Methyl[3-phthalimido-3-(3,4-dimethoxyphenyl)]propionat ist.

6. Verbindung gemäß Anspruch 4, welche Methyl[3-phthalimido-3-(3,4-diethoxyphenyl)]propionat ist.

7. Verbindung nach Anspruch 2, wobei R⁶ -CH₂- ist.

8. Verbindung nach Anspruch 7, wobei R⁵ *o*-Phenylen ist, unsubstituiert oder substituiert mit 1 bis 3 Substituenten, jeweils unabhängig voneinander ausgewählt aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 4 Kohlenstoffatomen, Alkoxy mit 1 bis 4 Kohlenstoffatomen oder Halogen.

9. Verbindung gemäß Anspruch 1, wobei R⁶ -CH₂- ist.

10. Verbindung nach Anspruch 9, wobei R⁵ *o*-Phenylen ist, unsubstituiert oder substituiert mit 1 bis 3 Substituenten, jeweils unabhängig voneinander ausgewählt aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 4 Kohlenstoffatomen, Alkoxy mit 1 bis 4 Kohlenstoffatomen oder Halogen.

11. Verbindung gemäß Anspruch 1, wobei:
R⁵ *o*-Phenylen ist;
R⁶-CO- ist;
R⁷ *(i)* Pyridyl, *(ii)* Phenyl, substituiert mit Cyano, Trifluormethyl, Carbethoxy, Carbomethoxy, Carbopropoxy, Acetyl, Carbamoyl, Acetoxy, Carboxy, Hydroxy, Alkyl mit 1 bis 10 Kohlenstoffatomen, Alkoxy mit 2 bis 10 Kohlenstoffatomen oder Halogen, *(iii)* Phenyl, substituiert mit 2 oder mehr Substituenten, jeweils unabhängig voneinander ausgewählt aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 10 Kohlenstoffatomen, Alkoxy mit 1 bis 10 Kohlenstoffatomen oder Halogen, *(iv)* Benzyl, substituiert mit 1 bis 3 Substituenten, ausgewählt aus der Gruppe, bestehend aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 4 Kohlenstoffatomen, Alkoxy mit 1 bis 4 Kohlenstoffatomen oder Halogen, *(v)* Naphthyl, *(vi)* Benzyloxy oder *(vii)* Imidazol-4-ylmethyl ist;
R¹²-OH ist; und
wobei n einen Wert von 1 hat.

12. Verbindung gemäß Anspruch 1, wobei:
R⁵ *o*-Phenylen ist;
R⁶ -CO- ist;
R⁷ *(i)* Pyridyl, *(ii)* Phenyl, substituiert mit Cyano, Trifluormethyl, Carbethoxy, Carbomethoxy, Carbopropoxy, Acetyl, Carbamoyl, Acetoxy, Carboxy, Hydroxy, Alkyl mit 1 bis 10 Kohlenstoffatomen, Alkoxy mit 2 bis 10 Kohlenstoffatomen oder Halogen, *(iii)* Phenyl, substituiert mit 2 oder mehr Substituenten, jeweils unabhängig voneinander ausgewählt aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 10 Kohlenstoffatomen, Alkoxy mit 1 bis 10 Kohlenstoffatomen oder Halogen, *(iv)* Benzyl, substituiert mit 1 bis 3 Substituenten, ausgewählt aus der Gruppe, bestehend aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 4 Kohlenstoffatomen, Alkoxy mit 1 bis 4 Kohlenstoffatomen oder Halogen, *(v)* Naphthyl, *(vi)* Benzyloxy oder *(vii)* Imidazol-4-ylmethyl ist;
R¹² Ethoxy ist; und
wobei n einen Wert von 1 hat.

13. Verbindung gemäß Anspruch 1, wobei:
R¹² 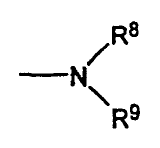 ist,
wobei R⁸ Wasserstoff oder Alkyl mit 1 bis 10 Kohlenstoffatomen ist;
R⁹ Wasserstoff, Alkyl mit 1 bis 10 Kohlenstoffatomen, -CH₂-Pyridyl, Benzyl, -COR¹⁰ oder -SO₂R¹⁰ ist, worin R¹⁰ Wasserstoff, Alkyl 1 bis 10 Kohlenstoffatomen oder Phenyl ist.

14. Verbindung gemäß Anspruch 13, wobei:
R⁵ *o*-Phenylen ist;
R⁶ -CO- ist;
R⁷ *(i)* Pyridyl, *(ii)* Phenyl, substituiert mit einem oder mehr Substituenten, jeweils unabhängig voneinander ausgewählt ist aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 10 Kohlenstoffatomen, Alkoxy mit 1 bis 10 Kohlenstoffatomen oder Halogen, *(iii)* Benzyl, substituiert mit 1 bis 3 Substituenten, ausgewählt aus der Gruppe, bestehend aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 4 Kohlenstoffatomen, Alkoxy mit 1 bis 4 Kohlenstoffatomen oder Halogen, *(iv)* Naphthyl, *(v)* Benzyloxy oder *(vi)* Imidazol-4-ylmethyl ist; und
wobei n einen Wert von 1 hat.

15. Verbindung gemäß Anspruch 14, welche 3-Phthalimido-3-(4-methoxyphenyl)propanamid ist.

16. Verbindung gemäß Anspruch 14, welche 3-Phthalimido-3-(3,4-dimethoxyphenyl)propanamid ist.

17. Verbindung gemäß Anspruch 14, welche 3-Phthalimido-3-(3,4-diethoxyphenyl)propanamid ist.

18. Verbindung gemäß Anspruch 1, wobei R⁵ aminosubstituiertes *o*-Phenylen ist.

19. Verbindung gemäß Anspruch 18, welche 3-(Aminophthalimido)-3-(3,4-dimethoxyphenyl)propanamid ist.

20. Verbindung gemäß Anspruch 18, welche 3-(Aminophthalimido)-3-(3,4-diethoxyphenyl)propanamid ist.

21. Verbindung gemäß Anspruch 18, welche Methyl[3-(aminophthalimido)-3-(3,4-dimethoxyphenyl)propionat ist.

22. Verbindung gemäß Anspruch 18, welche Methyl[3-(aminophthalimido)-3-(3,4-diethoxyphenyl)]propionat ist.

23. Verbindung gemäß einem der Ansprüche 1 bis 22 für die Verwendung in der Therapie.

24. Pharmazeutische Zusammensetzung, umfassend eine Menge einer Verbindung gemäß einem der Ansprüche 1 bis 22, die nach einmaliger oder mehrmaliger Dosierung zur Hemmung von TNFα wirksam ist.

25. Verwendung einer Verbindung in der Herstellung eines Medikaments für die Behandlung einer Kränkheit, die durch Hemmen der Bildung von oder Verminderung von TNFα-Spiegeln in einem Säuger behandelbar ist, wobei die Verbindung die Formel: 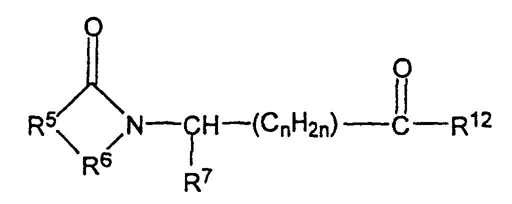 aufweist,
worin R⁵ *(i) o*-Phenylen, unsubstituiert oder substituiert mit 1 bis 3 Substituenten, jeweils unabhängig voneinander ausgewählt aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 4 Kohlenstoffatomen, Alkoxy mit 1 bis 4 Kohlenstoffatomen oder Halogen oder *(ii)* der zweiwertige Rest von Pyridin, Pyrrolidin, Imidizol, Naphthalin oder Thiophen ist, wobei die zweiwertigen Bindungen an vicinalen Ringkohlenstoffatomen sind;
R⁶ -CO-, -CH₂- oder -SO₂- ist;
R⁷ *(i)* geradkettiges, verzweigtes oder zyklisches Alkyl mit 1 bis 12 Kohlenstoffatomen, *(ii)* Pyridyl, *(iii)* Phenyl oder Phenyl, substituiert mit einem oder mehreren Substituenten, jeweils unabhängig voneinander ausgewählt aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 10 Kohlenstoffatomen, Alkoxy mit 1 bis 10 Kohlenstoffatomen oder Halogen, *(iv)* Benzyl, unsubstituiert oder substituiert mit einem bis drei Substituenten, ausgewählt aus der Gruppe, bestehend aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 4 Kohlenstoffatomen, Alkoxy mit 1 bis 4 Kohlenstoffatomen oder Halogen, *(v)* Naphthyl, *(vi)* Benzyloxy oder *(vii)* Imidazol-4-ylmethyl ist;
R¹² -OH, Alkoxy mit 1 bis 12 Kohlenstoffatomen, -O-CH₂-Pyridyl, -O-Benzyl oder 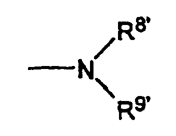 ist,
wobei n einen Wert von 0, 1, 2 oder 3 hat;
R⁸ Wasserstoff oder Alkyl mit 1 bis 10 Kohlenstoffatomen ist; und
R⁹ Wasserstoff, Alkyl mit 1 bis 10 Kohlenstoffatomen, -CH₂-Pyridyl, Benzyl, -COR¹⁰ oder -SO₂R¹⁰ ist, worin R¹⁰ Wasserstoff, Alkyl mit 1 bis 4 Kohlenstoffatomen oder Phenyl ist.

26. Verwendung einer Verbindung gemäß Anspruch 25, wobei R¹² 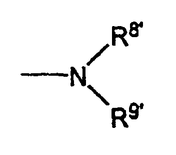 ist.

27. Verwendung einer Verbindung gemäß Anspruch 26, wobei in der Verbindung:
R⁵ *o*-Phenylen ist;
R⁶-CO- ist; und
wobei n einen Wert von 1 hat.

28. Verwendung einer Verbindung gemäß Anspruch 25, wobei in der Verbindung:
R⁵ *o*-Phenylen ist;
R⁶ -CO- ist;
R¹² Methoxy ist; und
wobei n einen Wert von 1 hat.

29. Verwendung einer Verbindung gemäß Anspruch 25, wobei in der Verbindung:
R⁵ *o*-Phenylen ist;
R⁶-CO- ist;
R¹²-OH ist; und
wobei n einen Wert von 1 hat.

30. Verwendung einer Verbindung gemäß Anspruch 25, wobei in der Verbindung:
R⁵ *o*-Phenylen ist;
R⁶-CO- ist;
R¹² Ethoxy ist; und
wobei n einen Wert von 1 hat.

31. Verwendung einer Verbindung gemäß einem der Ansprüche 1 bis 22 in der Herstellung eines Medikaments für die Verminderung von TNFα-Spiegeln in einem Säuger.

32. Verwendung einer Verbindung gemäß einem der Ansprüche 1 bis 22 in der Herstellung eines Medikaments für die Hemmung von TNFα-aktivierter Retrovirus-Replikation in Säugern.

## Revendications

1. Composé de formule : 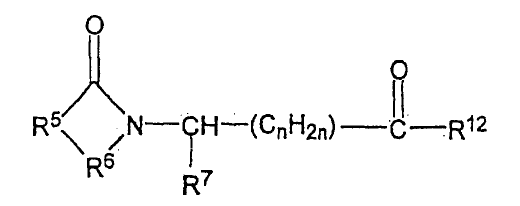 dans laquelle
R⁵ est *(i)* un groupe o-phénylène, non substitué ou substitué par 1 à 3 substituants choisis chacun indépendamment parmi les groupes nitro, cyano, trifluorométhyle, carbéthoxy, carbométhoxy, carbopropoxy, acétyle, carbamoyle, acétoxy, carboxy, hydroxy, amine, alkyle de 1 à 4 atomes de carbone, alcoxy de 1 à 4 atomes de carbone ou halogéno, ou bien *(ii)* le résidu divalent d'un groupe pyridine, pyrrolidine, imidazole, naphtalène ou thiophène, dans lequel les liaisons divalentes sont situées sur des atomes de carbone vicinaux du noyau;
R⁶ est -CO-, -CH₂- ou -SO₂- ;
R⁷ est *(i)* un groupe pyridyle, *(ii)* un groupe phényle substitué par un ou plusieurs substituants choisis chacun indépendamment les uns des autres parmi les groupes nitro, cyano, trifluorométhyle, carbéthoxy, carbométhoxy, carbopropoxy, acétyle, carbamoyle, acétoxy, carboxy, hydroxy, amino, alkyle de 1 à 10 atomes de carbone, alcoxy de 1 à 10 atomes de carbone ou halogéno, *(iii)* un groupe benzyle substitué par un à trois substituants choisis dans le groupe constitué par les groupes nitro, cyano, trifluorométhyle, carbéthoxy, carbométhoxy, carbopropoxy, acétyle, carbamoyle, acétoxy, carboxy, hydroxy, amino, alkyle de 1 à 4 atomes de carbone, alcoxy de 1 à 4 atomes de carbone ou halogéno, *(iv)* un groupe naphtyle, *(v)* un groupe benzyloxy ou *(vi)* un groupe imidazol-4-ylméthyle ;
R¹² est un groupe -OH, alcoxy de 1 à 12 atomes de carbone, -O-CH₂-pyridyle, -O-benzyle, ou 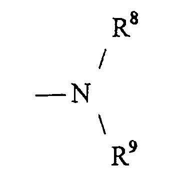 où n a une valeur de 0, 1, 2 ou 3 ;
R⁸ est un atome d'hydrogène ou un groupe alkyle de 1 à 10 atomes de carbone ; et
R⁹ est un atome d'hydrogène ou un groupe alkyle de 1 à 10 atomes de carbone, -CH₂-pyridyle, benzyle, -COR¹⁰ ou -SO₂R¹⁰, où R¹⁰ est un atome d'hydrogène ou un groupe alkyle de 1 à 4 atomes de carbone, ou phényle.

2. Composé selon la revendication 1 dans lequel R¹² est un groupe -O-méthyle.

3. Composé selon la revendication 2 dans lequel R⁵ est un groupe o-phénylène non substitué ou substitué par 1 à 3 substituants choisis chacun indépendamment parmi les groupes nitro, cyano, trifluorométhyle, carbéthoxy, carbométhoxy, carbopropoxy, acétyle, carbamoyle, acétoxy, carboxy, hydroxy, amino, alkyle de 1 à 4 atomes de carbone, alcoxy de 1 à 4 atomes de carbone ou halogéno.

4. Composé selon la revendication 3 dans lequel :
R⁵ est un groupe o-phénylène ;
R⁶ est -CO- ;
R⁷ est *(i)* un groupe pyridyle, *(ii)* un groupe phényle substitué par un groupe cyano, trifluorométhyle, carbéthoxy, carbométhoxy, carbopropoxy, acétyle, carbamoyle, acétoxy, carboxy, hydroxy, alkyle de 2 à 10 atomes de carbone, alcoxy de 1 à 10 atomes de carbone ou halogéno, *(iii)* un groupe phényle substitué par au moins 2 substituants choisis chacun indépendamment les uns des autres parmi les groupes nitro, cyano, trifluorométhyle, carbéthoxy, carbométhoxy, carbopropoxy, acétyle, carbamoyle, acétoxy, carboxy, hydroxy, amino, alkyle de 1 à 10 atomes de carbone, alcoxy de 1 à 10 atomes de carbone ou halogéno, *(iv)* un groupe benzyle substitué par 1 à 3 substituants choisis dans le groupe constitué par les groupes nitro, cyano, trifluorométhyle, carbéthoxy, carbométhoxy, carbopropoxy, acétyle, carbamoyle, acétoxy, carboxy, hydroxy, amino, alkyle de 1 à 4 atomes de carbone, alcoxy de 1 à 4 atomes de carbone ou halogéno, *(v)* un groupe naphtyle, *(vi)* un groupe benzyloxy ou *(vii)* un groupe imidazol-4-ylméthyle ; et
où n est égal à 1.

5. Composé selon la revendication 4 qui est le [3-phtalimido-3-(3,4-diméthoxyphényl)]propionate de méthyle.

6. Composé selon la revendication 4 qui est le [3-phtalimido-3-(3,4-diéthoxyphényl)]propionate de méthyle.

7. Composé selon la revendication 2 dans lequel R⁶ est -CH₂-.

8. Composé selon la revendication 7 dans lequel R⁵ est un groupe o-phénylène, non substitué ou substitué par 1 à 3 substituants choisis chacun indépendamment parmi les groupes nitro, cyano, trifluorométhyle, carbéthoxy, carbométhoxy, carbopropoxy, acétyle, carbamoyle, acétoxy, carboxy, hydroxy, amino, alkyle de 1 à 4 atomes de carbone, alcoxy de 1 à 4 atomes de carbone ou halogéno.

9. Composé selon la revendication 1 dans lequel R⁶ est -CH₂-.

10. Composé selon la revendication 9 dans lequel R⁵ est un groupe o-phénylène, non substitué ou substitué par 1 à 3 substituants choisis chacun indépendamment parmi les groupes nitro, cyano, trifluorométhyle, carbéthoxy, carbométhoxy, carbopropoxy, acétyle, carbamoyle, acétoxy, carboxy, hydroxy, amino, alkyle de 1 à 4 atomes de carbone, alcoxy de 1 à 4 atomes de carbone ou halogéno.

11. Composé selon la revendication 1 dans lequel:
R⁵ est un groupe o-phénylène ;
R⁶ est -CO- ;
R⁷ est *(i)* un groupe pyridyle, *(ii)* un groupe phényle substitué par un groupe cyano, trifluorométhyle, carbéthoxy, carbométhoxy, carbopropoxy, acétyle, carbamoyle, acétoxy, carboxy, hydroxy, amino, alkyle de 1 à 10 atomes de carbone, alcoxy de 2 à 10 atomes de carbone ou halogéno, *(iii)* un groupe phényle substitué par au moins 2 substituants choisis chacun indépendamment les uns des autres parmi les groupes nitro, cyano, trifluorométhyle, carbéthoxy, carbométhoxy, carbopropoxy, acétyle, carbamoyle, acétoxy, carboxy, hydroxy, amino, alkyle de 1 à 10 atomes de carbone, alcoxy de 1 à 10 atomes de carbone ou halogéno, *(iv)* un groupe benzyle substitué par 1 à 3 substituants choisis dans le groupe constitué par les groupes nitro, cyano, trifluorométhyle, carbéthoxy, carbométhoxy, carbopropoxy, acétyle, carbamoyle, acétoxy, carboxy, hydroxy, amino, alkyle de 1 à 4 atomes de carbone, alcoxy de 1 à 4 atomes de carbone ou halogéno, *(v)* un groupe naphtyle, *(vi)* un groupe benzyloxy ou *(vii)* un groupe imidazol-4-ylméthyle;
R¹² est -OH ; et
où n est égal à 1.

12. Composé selon la revendication 1 dans lequel :
R⁵ est un groupe o-phénylène ;
R⁶ est -CO- ;
R⁷ est *(i)* un groupe pyridyle, *(ii)* un groupe phényle substitué par un groupe cyano, trifluorométhyle, carbéthoxy, carbométhoxy, carbopropoxy, acétyle, carbamoyle, acétoxy, carboxy, hydroxy, alkyle de 1 à 10 atomes de carbone, alcoxy de 2 à 10 atomes de carbone ou halogéno, *(iii)* un groupe phényle substitué par au moins 2 substituants choisis chacun indépendamment les uns des autres parmi les groupes nitro, cyano, trifluorométhyle, carbéthoxy, carbométhoxy, carbopropoxy, acétyle, carbamoyle, acétoxy, carboxy, hydroxy, amino, alkyle de 1 à 10 atomes de carbone, alcoxy de 1 à 10 atomes de carbone, ou halogéno, *(iv)* un groupe benzyle substitué par 1 à 3 substituants choisis dans le groupe constitué par les groupes nitro, cyano, trifluorométhyle, carbéthoxy, carbométhoxy, carbopropoxy, acétyle, carbamoyle, acétoxy, carboxy, hydroxy, amino, alkyle de 1 à 4 atomes de carbone, alcoxy de 1 à 4 atomes de carbone, ou halogéno, *(v)* un groupe naphtyle, *(vi)* un groupe benzyloxy ou *(vii)* un groupe imidazol-4-ylméthyle ;
R¹² est un groupe éthoxy ; et
où n est égal à 1.

13. Composé selon la revendication 1 dans lequel: 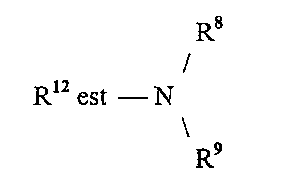 où R⁸ est un atome d'hydrogène ou un groupe alkyle de 1 à 10 atomes de carbone ;
R⁹ est un atome d'hydrogène ou un groupe alkyle de 1 à 10 atomes de carbone, -CH₂-pyridle, benzyle, -COR¹⁰ ou -SO₂R¹⁰, où R¹⁰ est un atome d'hydrogène ou un groupe alkyle de 1 à 10 atomes de carbone, ou phényle.

14. Composé selon la revendication 13 dans lequel :
R⁵ est un groupe o-phénylène ;
R⁶ est -CO- ;
R⁷ est *(i)* un groupe pyridyle, *(ii)* un groupe phényle substitué par un ou plusieurs substituants choisis chacun indépendamment les uns des autres parmi les groupes nitro, cyano, trifluorométhyle, carbéthoxy, carbométhoxy, carbopropoxy, acétyle, carbamoyle, acétoxy, carboxy, hydroxy, amino, alkyle de 1 à 10 atomes de carbone, alcoxy de 1 à 10 atomes de carbone ou halogéno, *(iii)* un groupe benzyle substitué par 1 à 3 substituants choisis dans le groupe constitué par les groupes nitro, cyano, trifluorométhyle, carbéthoxy, carbométhoxy, carbopropoxy, acétyle, carbamoyle, acétoxy, carboxy, hydroxy, amino, alkyle de 1 à 4 atomes de carbone, alcoxy de 1 à 4 atomes de carbone ou halogéno, *(iv)* un groupe naphtyle, *(v)* un groupe benzyloxy ou *(vi)* un groupe imidazol-4-ylméthyle ; et
où n est égal à 1.

15. Composé selon la revendication 14 qui est le 3-phtalimido-3-(4-méthoxyphényl)propanamide.

16. Composé selon la revendication 14 qui est le 3-phtalimido-3-(3,4-diméthoxyphényl)propanamide.

17. Composé selon la revendication 14 qui est le 3-phtalimido-3-(3,4-diéthoxyphényl)propanamide.

18. Composé selon la revendication 1 dans lequel R⁵ est un groupe o-phénylène substitué par un groupe amino.

19. Composé selon la revendication 18 qui est le 3-(aminophtalimido)-3-(3,4-diméthoxyphényl)propanamide.

20. Composé selon la revendication 18 qui est le 3-(aminophtalimido)-3-(3,4-diéthoxyphényl)propanamide.

21. Composé selon la revendication 18 qui est le [3-(aminophtalimido)-3-(3,4-diméthoxyphényl)]propionate de méthyle.

22. Composé selon la revendication 18 qui est le [3-(aminophtalimido)-3-(3,4-diéthoxyphényl)]propionate de méthyle.

23. Composé selon l'une quelconque des revendications 1 à 22 à utiliser en thérapeutique.

24. Composition pharmaceutique comprenant une quantité d'un composé selon l'une quelconque des revendications 1 à 22 efficace en une seule prise ou en plusieurs prises pour inhiber le TNFα.

25. Utilisation d'un composé dans la fabrication d'un médicament destiné au traitement d'une maladie susceptible d'être traitée par inhibition de la formation ou par réduction des taux de TNFα chez un mammifère, dans laquelle le composé a pour formule : 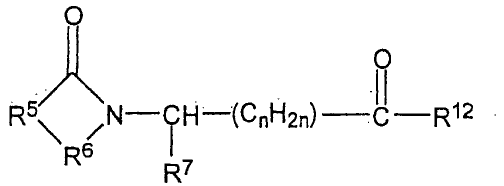 dans laquelle
R⁵ est *(i)* un groupe o-phénylène, non substitué ou substitué par 1 à 3 substituants choisis chacun indépendamment parmi les groupes nitro, cyano, trifluorométhyle, carbéthoxy, carbométhoxy, carbopropoxy, acétyle, carbamoyle, acétoxy, carboxy, hydroxy, amino, alkyle de 1 à 4 atomes de carbone, alcoxy de 1 à 4 atomes de carbone ou halogéno, ou bien *(ii)* le résidu divalent d'un groupe pyridine, pyrrolidine, imidazole, naphtalène ou thiophène, dans lequel les liaisons divalentes sont situées sur des atomes de carbone vicinaux du noyau;
R⁶ est -CO-, -CH₂- ou -SO₂- ;
R⁷ est *(i)* un groupe alkyle de 1 à 12 atomes de carbone, linéaire, ramifié ou cyclique, *(ii)* un groupe pyridyle, *(iii)* un groupe phényle ou phényle substitué par un ou plusieurs substituants choisis chacun indépendamment les uns des autres parmi les groupes nitro, cyano, trifluorométhyle, carbéthoxy, carbométhoxy, carbopropoxy, acétyle, carbamoyle, acétoxy, carboxy, hydroxy, amino, alkyle de 1 à 10 atomes de carbone, alcoxy de 1 à 10 atomes de carbone ou halogéno, *(iv)* un groupe benzyle non substitué ou substitué par un à trois substituants choisis dans le groupe constitué par les groupes nitro, cyano, trifluorométhyle, carbéthoxy, carbométhoxy, carbopropoxy, acétyle, carbamoyle, acétoxy, carboxy, hydroxy, amino, alkyle de 1 à 4 atomes de carbone, alcoxy de 1 à 4 atomes de carbone ou halogéno, *(v)* un groupe naphtyle, *(vi)* un groupe benzyloxy ou *(vii)* un groupe imidazol-4-ylméthyle ;
R¹² est un groupe -OH, alcoxy de 1 à 12 atomes de carbone, -O-CH₂-pyridyle, -O-benzyle, ou 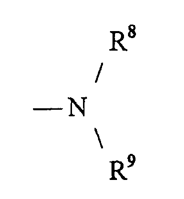 n a une valeur de 0, 1, 2 ou 3 ;
R⁸ est un atome d'hydrogène ou un groupe alkyle de 1 à 10 atomes de carbone ; et
R⁹ est un atome d'hydrogène ou un groupe alkyle de 1 à 10 atomes de carbone, -CH₂-pyridyle, benzyle, -COR¹⁰ ou -SO₂R¹⁰, où R¹⁰ est un atome d'hydrogène ou un groupe alkyle de 1 à 4 atomes de carbone, ou phényle.

26. Utilisation d'un composé selon la revendication 25 dans laquelle R¹² est

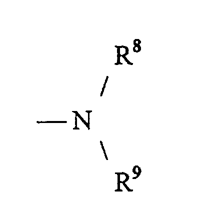

27. Utilisation d'un composé selon la revendication 26 dans laquelle, dans le composé :
R⁵ est un groupe o-phénylène ;
R⁶ est -CO- ; et
où n est égal à 1.

28. Utilisation d'un composé selon la revendication 25 dans laquelle, dans le composé :
R⁵ est un groupe o-phénylène ;
R⁶ est -CO- ;
R¹² est un groupe méthoxy ; et
où n est égal à 1.

29. Utilisation d'un composé selon la revendication 25 dans laquelle, dans le composé:
R⁵ est un groupe o-phénylène ;
R⁶ est -CO- ;
R¹² est -OH ; et
où n est égal à 1.

30. Utilisation d'un composé selon la revendication 25 dans laquelle, dans le composé :
R⁵ est un groupe o-phénylène ;
R⁶ est -CO- ;
R¹² est un groupe éthoxy ; et
où n est égal à 1.

31. Utilisation d'un composé selon l'une quelconque des revendications 1 à 22 dans la fabrication d'un médicament destiné à réduire les taux de TNFα chez un mammifère.

32. Utilisation d'un composé selon l'une quelconque des revendications 1 à 22 dans la fabrication d'un médicament destiné à inhiber la réplication d'un rétrovirus activée par le TNFα chez un mammifère.
